# EUROPEAN PATENT APPLICATION

(11) **EP 4 778 582 A2**
(43) Date of publication of application: **22.07.2026**
(21) Application number: 26180380.3
(22) Date of filing: 01.05.2020
(51) Int. Cl.: A61P 25/28

(54) **TREATMENT FOR SOD1 ASSOCIATED DISEASE**

(30) Priority: 01.05.2019 AU 2019901485
(62) Divisional of application: 20798482.4
(71) Applicant: The Perron Institute for Neurological and Translational Science Limited, Nedlands WA 6009 (AU)
(72) Inventor: WILTON, Stephen Donald, Murdoch 6150 (AU); FLETCHER, Susan, Murdoch 6150 (AU); FLYNN, Loren, Murdoch 6150 (AU); AKKARI, Patrick Anthony, Murdoch 6150 (AU)
(74) Representative: AWA Sweden AB

(57) **Abstract**

The present invention relates to antisense oligonucleotides that are complimentary to SOD1, leading to decreased expression of SOD1. Reduced expression of SOD1 is beneficial in medical disorders such as Amyotrophic Lateral Sclerosis.

## Description

### TEHCNICAL FIELD

The present invention relates to antisense oligonucleotides (AON) to induce alternative splicing through exon skipping in the superoxide dismutase (SOD1) gene. The invention provides methods to treat, prevent or ameliorate the effects of a disease associated with expression of the SOD1 gene by administration of AON and therapeutic compositions comprising AONs to the SOD1 gene.

### BACKGROUND ART

The following discussion of the background art is intended to facilitate an understanding of the present invention only. The discussion is not an acknowledgement or admission that any of the material referred to is or was part of the common general knowledge as at the priority date of the application.

The soluble SOD1 enzyme (also known as Cu/Zn superoxide dismutase) converts toxic superoxide free radicals within cells to non-toxic hydrogen peroxide (H202) (Fridovich, Annu. Rev. Biochem., 1995, 64, 97-112). The superoxide anion (O²⁻) is a potentially harmful cellular by-product that can cause oxidative stress. Neurons are particularly prone to oxidative stress due to their high metabolic needs, and therefore produce a large amount of superoxide radicals.

Mutations in the SOD1 gene have been implicated in amyotrophic lateral sclerosis (ALS). ALS is a fatal degenerative disease that affects motor neurons. ALS causes progressive muscular weakness and atrophy, leading to paralysis and death within about 3-5 years.

The only treatment options for ALS that is currently commercially available are riluzole, and edaravone (US and Japan only), which may extend life by about 2-3 months. All other available treatment options are palliative. About 5 - 10% of ALS cases are known to be caused by genetic factors. There is a tight genetic linkage between familial ALS and missense mutations in the SOD1 gene (Rosen, Nature, 1993, 362, 59-62), in that about 20% of the familial forms of ALS are caused by mutations to the SOD1 gene.

These mutations are likely to encode a gain of toxic function rather than a loss of SOD1 activities, as the complete absence of SOD1 in mice neither diminishes life nor provokes overt disease (Al-Chalabi and Leigh, Curr. Opin. Neural., 2000, 13, 397-405; Alisky and Davidson, Hum. Gene Ther., 2000, 11, 25 2315-2329). Mutated SOD1 has increased activity with abnormal substrates and reduced superoxide radical scavenging ability. To date, over 150 familial SOD1 mutations have been found (Cleveland, D and Rothstein, Nat. Rev. Neurosci, 2001, 2, 806-819; Andersen P, Curr. Neurol. Neurosci., 2006, 6, 1 37-46; Renton et al., Nat. Neurosci., 2014, 1, 17-23).

There are two models for mutant SOD1 toxicity (Cleveland and Liu, Nat. Med., 2000, 6, 1320-1321). The "oxidative hypothesis" ascribes toxicity to binding of aberrant substrates such as peroxynitrite or hydrogen peroxide which gain access to the catalytic copper ion through mutation-dependent loosening of the native SOD1 protein conformation (Cleveland and Liu, Nat. Med., 2000, 6, 1320-1321). A second potential mechanism for mutant SOD1 toxicity involves the misfolding and aggregation of mutant SOD1 proteins (Cleveland and Liu, Nat. Med., 2000, 6, 1320-1321). This hypothesis is supported by observations that murine models of SOD1 mutant-mediated disease feature prominent intracellular inclusions in motor neurons and, in some cases, in the astrocytes surrounding them as well (Bruijn et al., Science, 1998, 281, 1851-1854). Furthermore, Brujin *et al.* also demonstrate that neither elimination nor elevation of wild-type SOD1 was found to affect disease induced by mutant SOD1 in mice (Bruijn et al., Science, 1998, 281, 1851-1854).

Transgenic mice overexpressing mutant forms of the human SOD1 gene (for example SOD1^{G93A} mice) show most pathological features of ALS and are widely used in ALS preclinical studies (Gurney et al., Science, 1994, 264, 5166 1772-1775). Decreasing the expression of mutant SOD1 is therefore a rational strategy for treating SOD1-linked forms of ALS.

The accumulation of misfolded wildtype SOD1 protein aggregates have also been reported in ALS patients without SOD1 mutations, suggesting that SOD1 plays a central role in neurodegeneration. Misfolded wildtype SOD1 accumulation has been observed in post-mortem spinal cord tissue of ALS patients carrying mutations in *C9ORF72, FUS, KIP5, ALSIN, NEK1* and *VAPB* (Forsberg et al., J Neurol Neurosurg Psychiatry., 2019, 90, 8, 861-869) and in sporadic ALS patients (Forsberg et al., PLoS One, 2010, 5, 7, e11552; Rotunno et al., Front Cell Neurosci., 2013, 7 253). Misfolded wildtype SOD1 likely occurs via post-translational modifications, caused by oxidative stress and protein oxidation, and once misfolded, SOD1 likely sequesters, corrupts or exacerbates other key proteins into aggregates in a prion like fashion (Xu et al., Cell Death Dis., 2018, 9, 2, 67).

Genetic structural variations have been identified within ALS genes and surrounding gene regions and have been associated with sporadic disease risk and progression (Pytte et al., Neurol. Genet., 2020, 6, 2). Takuda *et al.* reported the presence of misfolded wildtype SOD1 in the CSF of sALS patients, detected by antibodies specific to misfolded SOD1, that were not evident in healthy controls (Takuda et al., Mol. Neurodegener. 2019, 14, 47).

US 2019/0256914 A1 describes a polyT structural variant within *SCAF4* and the *SOD1* gene region that associates with fALS risk.

Suppression of mutant SOD1 expression using siRNA has first proved significant therapeutic efficiency in SOD1-linked ALS mice. Raoul *et al.* showed that intraspinal injection of lentiviral vectors encoding short hairpin RNAs (shRNAs) to human SOD1 delayed disease onset and progression in SOD1^{G93A} mice (Raoul et al., Nat. Med. 2005, 11, 4 423-428). Independently, Ralph *et al.,* demonstrated that intramuscular injections of lentivirus mediating the expression of RNAi to the human SOD1, prevented neurodegeneration and extended survival in the same ALS mouse model, leading to a maximal 77% lifespan increase (Ralph et al., Nat. Med., 2005, 11, 4 429-433).

Continuous infusion of a modified AON into the brain ventricles was reported to allow efficient and widespread reduction of both SOD1 mRNA and protein levels throughout the brain and the spinal cord, significantly slowing disease progression in a rat model of ALS caused by the SOD1^{G93A} mutation (Smith et al., J Clin. Invest., 2006, 166, 2290-2296).

The expression of SOD1 in PC12 rat pheochromocytoma neuronal cells was inhibited by either of two 21-mer AONs targeting rat SOD1 nucleotides 54-74 and 497-517, leading to cellular apoptosis. The progression of cellular death was reversed by treatment with antioxidants (Troy and Shelanski, Proc. Natl. Acad. Sci. U. S. A., 1994, 91, 6384-6387).

Several potential AONs targeting SOD1 have been identified.

WO 94/19493 discloses oligonucleotide sequences encoding SOD1 and generally claims the use of an antisense DNA homolog of a gene encoding SOD1 in either mutant and wild-type forms in the preparation of a medicament for treating a patient with a disease.

Biferi et. al. (2017) discloses five anti-sense oligonucleotide sequences targeting SOD1 that were identified as inducing exon skipping to varying extents.

The use of double stranded nucleic acid molecules directed against SOD1 was also disclosed in WO 2017/007813. In particular, the use of sd-rxRNA with specific sequences was disclosed.

Further, in WO 2016/016449, four AONs targeting SOD1 were identified as inducing alternative splicing through exon skipping.

WO 2005/040180 and US 2017/0037410 A1 also describe 2'-*O*-Methoxyethyl gapmer AONs that induce RNase H-mediated SOD1 degradation.

None of these methods using AONs have as yet led to an effective commercially available ALS treatment.

Antisense technology is however emerging as an effective means for reducing the expression of specific gene products and may therefore prove to be uniquely useful in a number of therapeutic, diagnostic, and research applications for the modulation of SOD1 expression. Antisense technology can affect gene expression at a variety of different levels (transcription, splicing, stability, translation). However, the challenge with antisense technology is that it remains extremely difficult to identify specific AONs that have the desired effect *in vivo.*

Therefore, notwithstanding the extraordinary amount of research that has gone into identifying and developing an ALS treatment there still remains a need for a treatment for ALS. There is still no clear path to this treatment.

It is in the light of this background that the present invention has been developed. Particularly, the present invention seeks to provide a means for ameliorating ALS, thereby providing a disease treatment that to date has remained elusive.

### SUMMARY OF THE INVENTION

The present invention is directed to compounds, particularly AONs, which are targeted to a nucleic acid encoding SOD1. Embodiments of the present invention relate to AONs that are capable of binding to SOD1 pre-mRNA.

Broadly, according to one aspect of the invention, there is provided an isolated or purified AON targeted to a nucleic acid molecule encoding superoxide dismutase 1 pre-mRNA, wherein the AON has a nucleobase sequence that is:
a. selected from the list comprising SEQ ID NO: 1 to SEQ ID NO: 42 inclusive, or
b. a sequence that is of sufficient sequence complementarity to a target RNA to induce exon skipping wherein said sequence is complementary to at least 8 or more contiguous nucleobases in a target SOD1 pre-mRNA to which SEQ ID NO: 1 to SEQ ID NO: 42 inclusive also bind, and
c. wherein the AON inhibits the expression of human SOD1.

Preferably, the AON induces alternate splicing of SOD1 pre-mRNA through exon skipping. More preferably, the AON is a phosphorodiamidate morpholino oligomer and by way of example the invention is a nucleobase sequence selected from the list comprising SEQ ID NO: 1 to SEQ ID NO: 42 inclusive. Most preferably, the AON is a phosphorodiamidate morpholino oligomer and is conjugated with a peptide moiety.

The invention extends, according to a still further aspect thereof, to cDNA or cloned copies of the AON sequences of the invention, as well as to vectors containing one or more of the AON sequences of the invention. The invention extends further to cells containing such sequences and/or vectors.

There is also provided a method for inducing alternative splicing of SOD1 pre-mRNA, the method comprising the step of: providing one or more of the AON's as described herein and allowing the oligonucleotide to bind to a target nucleic acid site.

There is also provided a pharmaceutical, prophylactic, or therapeutic composition to treat, prevent or ameliorate the effects of a disease associated with mutations or misfolding in SOD1, the composition comprising one or more AONs as described herein; and one or more pharmaceutically acceptable carriers and/or diluents. Preferably, the disease associated with mutations in SOD1 is ALS.

There is also provided a pharmaceutical, prophylactic, or therapeutic composition to treat, prevent or ameliorate the effects of ALS, the composition comprising one or more AONs as described herein; and one or more pharmaceutically acceptable carriers and/or diluents.

There is also provided a method for treating, preventing or ameliorating the effects of a disease associated with mutations in SOD1 in a subject, the method comprising the step of: administering to the subject an effective amount of one or more AONs or pharmaceutical composition comprising one or more AONs as described herein.

There is also provided a method for treating, preventing or ameliorating the effects of a disease associated with misfolded SOD1 in a subject, the method comprising the step of: administering to the subject an effective amount of one or more AONs or pharmaceutical composition comprising one or more AONs as described herein.

There is also provided a method for treating, preventing or ameliorating the effects of ALS in a subject, the method comprising the step of: administering to the subject an effective amount of one or more AONs or pharmaceutical composition comprising one or more AONs as described herein.

There is also provided a method for treating, preventing or ameliorating the effects of ALS, in subjects identified by a biomarker, the method comprising the steps of:
a) testing a subject for the presence of a biomarker associated with ALS patients likely to respond to SOD1 suppression; and
b) if the subject is found to express the biomarker, administering to the subject an effective amount of one or more AONs or pharmaceutical composition comprising one or more AONs as described herein.

Preferably, the biomarker is a genetic structural variant within the *SOD1* gene region.

There is also provided the use of purified and isolated AONs as described herein, for the manufacture of a medicament to treat, prevent or ameliorate the effects of a disease associated with mutations or misfolding in SOD1.

There is also provided the use of purified and isolated AONs as described herein, for the manufacture of a medicament to treat, prevent or ameliorate the effects of ALS.

There is also provided herein the use of purified and isolated AONs as described herein, to treat, prevent or ameliorate the effects of a disease associated with mutations or misfolding in SOD1.

There is also provided herein the use of purified and isolated AONs as described herein, to treat, prevent or ameliorate the effects of ALS.

There is also provided herein a kit to treat, prevent or ameliorate the effects of a disease associated with mutations or misfolding in SOD1 in a subject, which kit comprises at least an AON as described herein, packaged in a suitable container, together with instructions for its use.

There is also provided herein a kit to treat, prevent or ameliorate the effects of ALS in a subject, which kit comprises at least an AON as described herein, packaged in a suitable container, together with instructions for its use.

Further features of the present invention are more fully described in the following description of several non-limiting embodiments thereof. This description is included solely for the purposes of exemplifying the present invention. It should not be understood as a restriction on the broad summary, disclosure or description of the invention as set out above.

### Brief Description of Drawings

The following description is provided with reference to the following accompanying drawings.
**Figure 1** shows RT-PCR analysis of the SOD1 transcript following transfection of 2'OMethyl AONs designed to induce SOD1 exon 2, 3 or 4 skipping.
**Figure 2** shows RT-PCR analysis of SOD1 transcripts following sequence optimisation and transfection of 2'OMethyl AONs.
**Figure 3** shows RT-PCR analysis of SOD1 transcripts following further exon 2, 3 and 4 targeting AON optimisation and transfection.
**Figure 4** shows the RT-PCR analysis of SOD1 transcripts following PMO transfection with AON sequences AON 6 and AON 8 by nucleofection.
**Figure 5** shows SOD1 transcript and protein analysis following PMO nucleofection with AON sequences AON 6 and AON 8.
**Figure 6** shows *SOD1* protein analysis following PMO nucleofection with AON sequences AON 6 and AON 8 in SOD1 patient fibroblasts.
**Figure 7** shows SOD1 transcript and protein analysis following PMO electroporation with AON 6 and AON 8 into SOD1-linked patient induced pluripotent stem cell (iPSC) derived motor neurons.
**Figure 8** shows the effect of SOD1 protein knockdown on lactate dehydrogenase (LDH) levels as a marker of cell viability in *SOD1*^{A4V} iPSC-derived motor neurons.
**Figure 9** shows the effect of SOD1 protein suppression on intracellular levels of reactive oxygen species (ROS) following hydrogen peroxide injury in PMO transfected (50 and 25 µM) *SOD1*^{D90A} fibroblasts.
**Figure 10** shows a comparison of the effect of AON 8 PMO and an RNase H phosphorothioate AON on SOD1 protein levels and cellular toxicity following electroporation into *SOD1*^{D90A} fibroblasts.
**Figure 11** shows SOD1 transcript and protein analysis from lysed brain tissue following the *in vivo* evaluation of SOD1 PMOs (AON 6 and AON 8) in transgenic *SOD1*^{G93A} mice.
**Figure 12** shows a diagram of the SOD1 transcript and the binding locations of the AONs in the present invention.

### DETAILED DESCRIPTION

The present invention provides a prophylactic or therapeutic method for ameliorating the symptoms of ALS using AON therapy. More specifically, the invention provides isolated or purified antisense oligonucleotides (AONs) that target to a nucleic acid molecule encoding superoxide dismutase 1 pre-mRNA, wherein the AON has a nucleobase sequence that is:
a. selected from the list comprising SEQ ID NO: 1 to SEQ ID NO: 42 inclusive, or
b. a sequence that is of sufficient sequence complementarity to a target RNA to induce exon skipping wherein said sequence is complementary to at least 8 or more contiguous nucleobases in a target *SOD1* pre-mRNA to which SEQ ID NO: 1 to SEQ ID NO: 42 inclusive also bind, and
c. wherein the AON inhibits the expression of human SOD1.

Preferably, the AON induces alternate splicing of SOD1 pre-mRNA through exon skipping. More preferably, the AON is a phosphorodiamidate morpholino oligomer and by way of example the invention is a nucleobase sequence selected from the list comprising SEQ ID NO: 1 to SEQ ID NO: 42 inclusive.

For convenience, the following sections generally outline the various meanings of the terms used herein. Following this discussion, general aspects regarding compositions, use of medicaments and methods of the invention are discussed, followed by specific examples demonstrating the properties of various embodiments of the invention and how they can be employed.

### 1. Definitions

The meaning of certain terms and phrases used in the specification, examples, and appended claims, are provided below. If there is an apparent discrepancy between the usage of a term in the art and its definition provided herein, the definition provided within the specification shall prevail.

Those skilled in the art will appreciate that the invention described herein is susceptible to variations and modifications other than those specifically described. The invention includes all such variations and modifications. The invention also includes all of the steps, features, formulations and compounds referred to or indicated in the specification, individually or collectively and any and all combinations or any two or more of the steps or features.

Each document, reference, patent application or patent cited in this text is expressly incorporated herein in their entirety by reference, which means that it should be read and considered by the reader as part of this text. That the document, reference, patent application or patent cited in this text is not repeated in this text is merely for reasons of conciseness. None of the cited material or the information contained in that material should, however be understood to be common general knowledge.

Manufacturer's instructions, descriptions, product specifications, and product sheets for any products mentioned herein or in any document incorporated by reference herein, are hereby incorporated herein by reference, and can be employed in the practice of the invention.

The present invention is not to be limited in scope by any of the specific embodiments described herein. These embodiments are intended for the purpose of exemplification only. Functionally equivalent products, formulations and methods are clearly within the scope of the invention as described herein.

Other than in the operating examples, or where otherwise indicated, all numbers expressing quantities of ingredients or reaction conditions used herein should be understood as modified in all instances by the term "about." The term "about" when used in connection with percentages can mean ±1%.

The invention described herein may include one or more range of values (e.g. size, concentration etc.). A range of values will be understood to include all values within the range, including the values defining the range, and values adjacent to the range which lead to the same or substantially the same outcome as the values immediately adjacent to that value which defines the boundary to the range. For example, a person skilled in the field will understand that a 10% variation in upper or lower limits of a range can be totally appropriate and is encompassed by the invention. More particularly, the variation in upper or lower limits of a range will be 5% or as is commonly recognised in the art, whichever is greater.

In this application, the use of the singular also includes the plural unless specifically stated otherwise. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise. Also, the use of the term "portion" can include part of a moiety or the entire moiety.

Throughout this specification, unless the context requires otherwise, the word "comprise" or variations such as "comprises" or "comprising", will be understood to imply the inclusion of a stated integer or group of integers but not the exclusion of any other integer or group of integers.

As used herein, the term "administer" refers to the placement of a composition into a subject by a method or route which results in at least partial localization of the composition at its desired site of action such that desired effect is produced. A compound or composition described herein can be administered by any appropriate route known in the art including, but not limited to, oral or parenteral routes, including intravenous, intramuscular, subcutaneous, transdermal, airway (aerosol), pulmonary, nasal, rectal, and topical (including buccal and sublingual) administration.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

Features of the invention will now be discussed with reference to the following non-limiting description and examples.

### 2. Embodiments

Embodiments of the present invention relate generally to improved antisense compounds, and methods or use thereof, which are specifically designed to supress the expression of the *SOD1* gene. Mutations and misfolding in the *SOD1* gene have been implicated in diseases such as ALS.

Without being bound by theory, the present invention is based on the understanding that suppressing the expression of *SOD1* in patients suffering from ALS may have the effect of improving survival of these patients. This is because the mutations in the SOD1 gene in ALS patients have been shown to result in a gain of toxic function. Therefore, the suppression of the SOD1 gene is hypothesised to result in improved survival of ALS patients. The ALS patients that can benefit from this therapy may have mutations or misfolding in the SOD1 gene. However, ALS patients that do not exhibit SOD1 mutations or misfolding may also respond to treatment supressing the SOD1 gene.

Preferably, the present invention provides AONs that are capable of binding a selected target on a nucleic acid molecule encoding *SOD1* so as to induce exon skipping, and consequently result in alternative splicing of the gene, no longer encoding a complete SOD1 enzyme. The alternatively spliced gene will therefore result in the downregulation of the expression of the full *SOD1* enzyme.

### A. Antisense Oligonucleotides

This invention provides one or more isolated or purified AONs that target a nucleic acid molecule encoding *SOD1* pre-mRNA, wherein the AON has a nucleobase sequence selected from the list comprising SEQ ID NO: 1 to SEQ ID NO: 42 inclusive (as set out in Tables 1 and 2, below) and wherein the AON inhibits the expression of human *SOD1.* Preferably, the AON induces alternate splicing of *SOD1* pre-mRNA through exon skipping. More preferably, the AON is a phosphorodiamidate morpholino oligomer.

More generally, the invention provides isolated or purified antisense oligonucleotides (AONs) that target to a nucleic acid molecule encoding superoxide dismutase 1 pre-mRNA, wherein the AON has a nucleobase sequence that is:
a. selected from the list comprising SEQ ID NO: 1 to SEQ ID NO: 42 inclusive, or
b. a sequence that is of sufficient sequence complementarity to a target RNA to induce exon skipping wherein said sequence is complementary to at least 8 or more contiguous nucleobases in a target SOD1 pre-mRNA to which SEQ ID NO: 1 to SEQ ID NO: 42 inclusive also bind, and
c. wherein the AON inhibits the expression of human *SOD1.*

Preferably, the AON induces alternate splicing of SOD1 pre-mRNA through exon skipping. More preferably, the AON is a phosphorodiamidate morpholino oligomer.

**Table 1**

| **SEQ ID NO** | **Co-ordinates** | **Sequence 5'→3'** |
|---|---|---|
| 1 | *SOD1* H2A (-12+13) | GUCCAUUACUUUCCUUUAAGAAAAG |
| 2 | *SOD1* H2A (+18+43) | GUCCUUUAAUGCUUCCCCACACCUU |
| 3 | *SOD1* H2A (+35+57) | CAGGCUUCAGUCAGUCCUUUAA |
| 4 | *SOD1* H2A (+54+79) | ACUCAUGAACAUGGAAUCCAUGCAGG |
| 5 | *SOD1* H2D (+13-08) | ACACCCACCUGCUGUAUUAUC |
| 6 | *SOD1* H2D (+16-09) | AACACCCACCUGCUGUAUUAUCUCC |
| 7 | *SOD1* H3A (+05+27) | UAAAGUGAGGACCUGCACUGGUA |
| 8 | *SOD1* H3A (+37+62) | AUCCUUUGGCCCACCGUGUUUUCUG |
| 9 | *SOD1* H3A (+42+63) | CAUCCUUUGGCCCACCGUGUU |
| 10 | *SOD1* H3D (+20-05) | GUUACCUCUCUUCAUCCUUUGGCCC |
| 11 | *SOD1* H4A (-16+09) | CCAACAUGCCUAAUAAUGAAAAAGC |
| 12 | *SOD1* H4A (+15+37) | UUUGUCAGCAGUCACAUUGCCCA |
| 13 | *SOD1* H4A (+38+62) | CAAUAGACACAUCGGCCACACCAUC |
| 14 | *SOD1* H4A (+79+101) | UGAUGCAAUGGUCUCCUGAGAGU |
| 15 | *SOD1* H4D (+06-19) | UCCUUUUAUGAAAACUUACCACCAG |
| 16 | *SOD1* H1A(+182+206) | UGAUGAUGCCCUGCACUGGGCCGUC |
| 17 | *SOD1* H1D(+20-05) | CUUGCCUUCUGCUCGAAAUUGAUGA |
| 18 | *SOD1* H2D(+21-04) | CCACCUGCUGUAUUAUCUCCAAACU |
| 19 | *SOD1* H3A(+32+57) | UUGGCCCACCGUGUUUUCUGGAUAG |
| 20 | *SOD1* H4A(-05+24) | AUUAGGCAUGUUGGAGACUUGGGCA |
| 21 | *SOD1* H4A(+28+52) | UGCUGACAAAGAUGGUGUGGCCGAU |

| | | |
|---|---|---|
| *Reverse complement sequence shown 5-3'. The reference point (0) set at first base of the 5' and 3' splice sites; hence* "+" *refers to nucleotides binding within the exon and* "-" *indicates nucleotides binding within the intron.* | | |

The AONs of the invention are designed to complement suitable sequences within the human *SOD1* (h*SOD1*) pre-mRNA which are required for correct splicing of the targeted exon, thereby blocking splicing reactions that would incorporate the targeted exon into mature mRNA.

The terms "antisense oligomer" and "antisense compound" and "antisense oligonucleotide" or "AON" are used interchangeably and refer to a linear sequence of cyclic subunits, each bearing a base-pairing moiety, linked by intersubunit linkages that allow the base-pairing moieties to hybridize to a target sequence in a nucleic acid (typically an RNA) by Watson-Crick base pairing, to form a nucleic acid:oligomer heteroduplex within the target sequence. The cyclic subunits are based on ribose or another pentose sugar or, in a preferred embodiment, a morpholino group (see description of morpholino oligomers below). The oligomer may have exact or near sequence complementarity to the target sequence; variations in sequence near the termini of an oligomer are generally preferable to variations in the interior. Also contemplated are peptide nucleic acids (PNAs), locked nucleic acids (LNAs), and 2'-O-Methyl oligonucleotides, among other antisense agents known in the art.

By "isolated" it is meant material that is substantially or essentially free from components that normally accompany it in its native state. For example, an "isolated polynucleotide" or "isolated oligonucleotide," as used herein, may refer to a polynucleotide that has been purified or removed from the sequences that flank it in a naturally-occurring state, e.g., a DNA fragment that is removed from the sequences that are adjacent to the fragment in the genome. The term "isolating" as it relates to cells refers to the purification of cells (e.g., fibroblasts, lymphoblasts) from a source subject (e.g., a subject with a polynucleotide repeat disease). In the context of mRNA or protein, "isolating" refers to the recovery of mRNA or protein from a source, e.g., cells.

An AON can be said to be "directed to" or "targeted against" a target sequence with which it hybridizes. In certain embodiments, the target sequence includes a region including the polyadenylation site and surrounding regions. The target sequence is typically a region including an AUG start codon of an mRNA, a Translation Suppressing Oligomer, or splice site of a pre-processed mRNA, a Splice Suppressing Oligomer (SSO). The target sequence for a splice site may include an mRNA sequence having its 5' end 1 to about 25 base pairs downstream of a normal splice acceptor junction in a pre-processed mRNA. A preferred target sequence is any region of a pre-processed mRNA that includes a splice site or is contained entirely within an exon coding sequence or spans a splice acceptor or donor site. An oligomer is more generally said to be "targeted against" a biologically relevant target, such as a protein, virus, or bacteria, when it is targeted against the nucleic acid of the target in the manner described above.

As used herein, "sufficient length" or "sufficient sequence complementarity" refers to an AON that is complementary to at least 8, more typically 8-30, contiguous nucleobases in a target SOD1 pre-mRNA. In some embodiments, an antisense of sufficient length includes at least 8, 9, 10, 11, 12, 13, 14, or 15 contiguous nucleobases in the target *SOD1* pre-mRNA. In other embodiments an antisense of sufficient length includes at least 16, 17, 18, 19, 20, 21, 22, 23, 24, or 25 contiguous nucleobases in the target *SOD1* pre-mRNA. Preferably, an oligonucleotide of sufficient length is from about 10 to about 50 nucleotides in length, including oligonucleotides of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 and 40 or more nucleotides. In one embodiment, an oligonucleotide of sufficient length is from 10 to about 30 nucleotides in length. In another embodiment, an oligonucleotide of sufficient length is from 15 to about 25 nucleotides in length. In yet another embodiment, an oligonucleotide of sufficient length is from 20 to 30, or 20 to 50, nucleotides in length. In yet another embodiment, an oligonucleotide of sufficient length is from 22 to 28, 25 to 28, 24 to 29 or 25 to 30 nucleotides in length.

In certain embodiments, the AON has sufficient sequence complementarity to a target RNA to block a region of a target RNA (e.g., pre-mRNA) in an effective manner. In exemplary embodiments, such blocking of SOD1 pre-RNA serves to induce exon skipping. In some embodiments, the target RNA is target pre-RNA (e.g., SOD1 gene pre-RNA).

In certain embodiments, AONs may be 100% complementary to the target sequence, or may include mismatches, e.g., to accommodate variants, as long as a heteroduplex formed between the oligonucleotide and target sequence is sufficiently stable to withstand the action of cellular nucleases and other modes of degradation which may occur in vivo. Hence, certain oligonucleotides may have about or at least about 70% sequence complementarity, e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence complementarity, between the oligonucleotide and the target sequence.

Mismatches, if present, are typically less destabilizing toward the end regions of the hybrid duplex than in the middle. The number of mismatches allowed will depend on the length of the oligonucleotide, the percentage of G:C base pairs in the duplex, and the position of the mismatch(es) in the duplex, according to well understood principles of duplex stability. Although such an AON is not necessarily 100% complementary to the target sequence, it is effective to stably and specifically bind to the target sequence, such that cleavage factor binding to the target pre-RNA is modulated.

The stability of the duplex formed between an AON and a target sequence is a function of the binding Tm and the susceptibility of the duplex to cellular enzymatic cleavage. The Tm of an oligonucleotide with respect to complementary-sequence RNA may be measured by conventional methods, such as those described by Hames et al., Nucleic Acid Hybridization, IRL Press, (1985), 107-108 or as described in Miyada C. G. and Wallace R. B., (1987), Methods Enzymol. 154, 94-107. In certain embodiments, AONs may have a binding Tm, with respect to a complementary-sequence RNA, of greater than body temperature and preferably greater than about 45°C or 50°C. Tm's in the range 60-80°C or greater are also included.

Additional examples of variants include AONs having about or at least about 70% sequence identity or homology, e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity or homology, over the entire length of any of SEQ ID NOS: 1-42. In respect of AON 6 (SEQ ID NO: 6) , in some embodiments, the AON of the invention exhibits 100% sequence identity or homology in respect of the first nine nucleotides AACACCCAC (SEQ ID NO: 43) and at least 70% sequence identity or homology in respect of nucleotides 10 to 25 CUGCUGUAUUAUCUCC (SEQ ID NO: 44). In respect of AON 27 (SEQ ID NO: 27), the AON of the invention exhibits 100% sequence identity or homology in respect of the first nine nucleotides AACACCCAC (SEQ ID NO: 43) and at least 70% sequence identity or homology in respect of nucleotides 10 to 25 CTGCTGTATTATCTCC (SEQ ID NO: 45).

More specifically, there is provided an AON capable of binding to a selected target site to induce exon skipping in a SOD1 gene transcript or part thereof. In an aspect, the AON induces skipping of exon 1, 2, 3 or 4 in a SOD1 gene. Preferably, the AON induces skipping of exon 2 or 3. Most preferably, the AON induces skipping of exon 3.

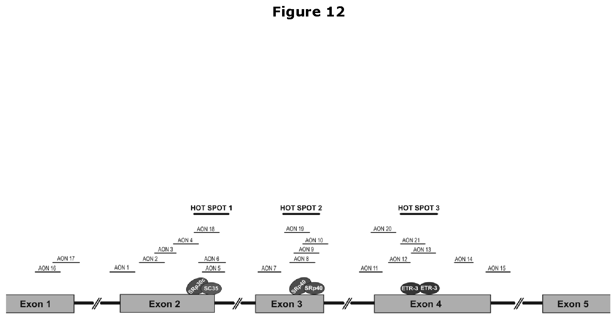

The location on pre-mRNA SOD1 to which an AON binds is relevant to its ability to induce exon skipping. In an aspect, the AON binds to a strongly predicted exonic splicing enhancer binding region, indicated as "hotspots" in Figure 12.

The AON is preferably selected from those provided in Table 1 or Table 2. For example, the AON used in the present invention is chosen from the list comprising SEQ ID NO:6, 8, 13, 27, 29, or 34. More preferably, the AON used in the present invention is SEQ ID NO: 8 or 29. Most preferably, the AON used in the present invention is a phosphorodiamidate morpholino oligomer of SEQ ID NO: 8 or 29.

### B. Methods of Use

The invention further provides a method of inducing alternative splicing of SOD1 pre-mRNA, the method comprising the steps of:
(a) providing one or more of the AONs as described herein and
(b) allowing the oligomer(s) to bind to a target nucleic acid site.

More specifically, the AON may be selected from those set forth in Table 1 or Table 2. The sequences are preferably selected from the group consisting of any one or more of SEQ ID Nos: 1-42, and combinations or cocktails thereof. This includes sequences which can hybridise to such sequences under stringent hybridisation conditions, sequences complementary thereto, sequences containing modified bases, modified backbones, and functional truncations or extensions thereof which possess or modulate RNA processing activity in a *SOD1* gene transcript.

Preferably, the AON used in the present invention is chosen from the list comprising SEQ ID NO:6, 8, 13, 27, 29, or 34. Most preferably, the AON used in the present invention is SEQ ID NO: 8 or 29.

The AONs used in the present method induce alternative splicing of SOD1 pre-mRNA. In an aspect, the AON induces alternative splicing through inducing exon skipping in *SOD1* pre-mRNA. Preferably, the AON induces skipping of exon 1, 2, 3 or 4 in a SOD1 gene. Preferably, the AON induces skipping of exon 2 or 3. Most preferably, the AON induces skipping of exon 3.

### Target Sequence and Selective Hybridisation

The oligomer and the DNA, cDNA or RNA are complementary to each other when a sufficient number of corresponding positions in each molecule are occupied by nucleotides which can hydrogen bond with each other. Thus, "specifically hybridisable" and "complementary" are terms which are used to indicate a sufficient degree of complementarity or pairing such that stable and specific binding occurs between the oligomer and the DNA, cDNA or RNA target. It is understood in the art that the sequence of an AON need not be 100% complementary to that of its target sequence to be specifically hybridisable. An AON is specifically hybridisable when binding of the compound to the target DNA or RNA molecule interferes with the normal function of the target DNA or RNA product, and there is a sufficient degree of complementarity to avoid non-specific binding of the AON to non-target sequences under conditions in which specific binding is desired, i.e., under physiological conditions in the case of in vivo assays or therapeutic treatment, and in the case of in vitro assays, under conditions in which the assays are performed.

Selective hybridisation may be under low, moderate or high stringency conditions, but is preferably under high stringency. Those skilled in the art will recognise that the stringency of hybridisation will be affected by such conditions as salt concentration, temperature, or organic solvents, in addition to the base composition, length of the complementary strands and the number of nucleotide base mismatches between the hybridising nucleic acids. Stringent temperature conditions will generally include temperatures in excess of 30°C, typically in excess of 37°C, and preferably in excess of 45°C, preferably at least 50°C, and typically 60°C-80°C or higher. Stringent salt conditions will ordinarily be less than 1000 mM, typically less than 500 mM, and preferably less than 200 mM. However, the combination of parameters is much more important than the measure of any single parameter. An example of stringent hybridisation conditions is 65°C and 0.1 x SSC (1 x SSC = 0.15 M NaCl, 0.015 M sodium citrate pH 7.0). Thus, the AONs of the present invention may include oligomers that selectively hybridise to the sequences provided in Table 1 or Table 2.

At a given ionic strength and pH, the Tm is the temperature at which 50% of a target sequence hybridizes to a complementary polynucleotide. Such hybridization may occur with "near" or "substantial" complementarity of the AON to the target sequence, as well as with exact complementarity.

Typically, selective hybridisation will occur when there is at least about 55% identity over a stretch of at least about 14 nucleotides, preferably at least about 65%, more preferably at least about 75% and most preferably at least about 90%, 95%, 98% or 99% identity with the nucleotides of the antisense oligomer. The length of homology comparison, as described, may be over longer stretches and in certain embodiments will often be over a stretch of at least about nine nucleotides, usually at least about 12 nucleotides, more usually at least about 20, often at least about 21, 22, 23 or 24 nucleotides, at least about 25, 26, 27 or 28 nucleotides, at least about 29, 30, 31 or 32 nucleotides, at least about 36 or more nucleotides.

Thus, the AON sequences of the invention preferably have at least 75%, more preferably at least 85%, more preferably at least 86, 87, 88, 89 or 90% homology to the sequences shown in the sequence listings herein. More preferably there is at least 91, 92, 93 94, or 95%, more preferably at least 96, 97, 98% or 99%, homology. Generally, the shorter the length of the antisense oligomer, the greater the homology required to obtain selective hybridisation. Consequently, where an AON of the invention consists of less than about 30 nucleotides, it is preferred that the percentage identity is greater than 75%, preferably greater than 85, 86, 87, 88, 89, 90, 91, 92, 93, 94, 95%, 96, 97, 98% or 99% compared with the AONs set out in the sequence listings herein. In respect of SEQ ID NO:6, in some embodiments, the AON of the invention exhibits 100% sequence homology in respect of the first nine nucleotides AACACCCAC (SEQ ID NO: 43) and at least 70% sequence homology in respect of nucleotides 10 to 25 CUGCUGUAUUAUCUCC (SEQ ID NO: 44). In respect of AON 27, the AON of the invention exhibits 100% sequence identity or homology in respect of the first nine nucleotides AACACCCAC (SEQ ID NO: 43) and at least 70% sequence identity or homology in respect of nucleotides 10 to 25 CTGCTGTATTATCTCC (SEQ ID NO: 45). Nucleotide homology comparisons may be conducted by sequence comparison programs such as the GCG Wisconsin Bestfit program or GAP (Deveraux et al., 1984, Nucleic Acids Research 12, 387-395). In this way sequences of a similar or substantially different length to those cited herein could be compared by insertion of gaps into the alignment, such gaps being determined, for example, by the comparison algorithm used by GAP.

The AON of the present invention may have regions of reduced homology, and regions of exact homology with the target sequence. It is not necessary for an oligomer to have exact homology for its entire length. For example, the oligomer may have continuous stretches of at least 4 or 5 bases that are identical to the target sequence, preferably continuous stretches of at least 6 or 7 bases that are identical to the target sequence, more preferably continuous stretches of at least 8 or 9 bases that are identical to the target sequence. The oligomer may have stretches of at least 10, 11, 12, 13, 14, 15, 16, 17, 18, 19, 20, 21, 22, 23, 24, 25 or 26 bases that are identical to the target sequence. The remaining stretches of oligomer sequence may be intermittently identical with the target sequence; for example, the remaining sequence may have an identical base, followed by a non-identical base, followed by an identical base. Alternatively (or as well) the oligomer sequence may have several stretches of identical sequence (for example 3, 4, 5 or 6 bases) interspersed with stretches of less than perfect homology. Such sequence mismatches will preferably have no or very little loss of cleavage modifying activity.

### Physiological Response

In an aspect, the method of the present invention induces a physiological response in a subject. Preferably, the method reduces the expression of *SOD1.*

The term "modulate" or "modulates" includes to "increase" or "decrease" one or more quantifiable parameters, optionally by a defined and/or statistically significant amount. The terms "increase" or "increasing," "enhance" or "enhancing," or "stimulate" or "stimulating" refer generally to the ability of one or AONs or compositions to produce or cause a greater physiological response (i.e., downstream effects) in a cell or a subject relative to the response caused by either no AON or a control compound.

By "enhance" or "enhancing," or "increase" or "increasing," or "stimulate" or "stimulating," refers generally to the ability of one or antisense compounds or compositions to produce or cause a greater physiological response (i.e., downstream effects) in a cell or a subject, as compared to the response caused by either no antisense compound or a control compound. An "increased" or "enhanced" amount is typically a "statistically significant" amount, and may include an increase that is 1.1, 1.2, 2, 3, 4, 5, 6, 7, 8, 9, 10, 15, 20, 30, 40, 50 or more times (e.g., 500, 1000 times) (including all integers and decimal points in between and above 1), e.g., 1.5, 1.6, 1.7, 1.8, etc.) the amount produced by no antisense compound (the absence of an agent) or a control compound.

The terms "decreasing" or "decrease" refer generally to the ability of one or AONs or compositions to produce or cause a reduced physiological response (i.e., downstream effects) in a cell or a subject relative to the response caused by either no AON or a control compound. The term "reduce" or "inhibit" may relate generally to the ability of one or more antisense compounds of the invention to "decrease" a relevant physiological or cellular response, such as a symptom of a disease or condition described herein, as measured according to routine techniques in the diagnostic art. Relevant physiological or cellular responses (in vivo or in vitro) will be apparent to persons skilled in the art and may include reductions in the symptoms or pathology of a SOD1 related condition. A "decrease" in a response may be statistically significant as compared to the response produced by no antisense compound or a control composition, and may include a 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% decrease, including all integers in between.

Relevant physiological or cellular responses (*in vivo* or *in vitro*) will be apparent to persons skilled in the art and may include decreases in the amount of SOD1 expression. An "increased" or "enhanced" amount is typically a statistically significant amount, and may include an increase that is 1.1, 1.2, 2, 3, 4, 5, 6, 7, 8, 9, 10, 15, 20, 30, 40, 50 or more times (e.g., 500, 1000 times) (including all integers and decimal points in between and above 1, e.g., 1.5, 1.6, 1.7. 1.8) the amount produced by no AON (the absence of an agent) or a control compound. The term "reduce" or "inhibit" may relate generally to the ability of one or more AONs or compositions to "decrease" a relevant physiological or cellular response, such as a symptom of a disease or condition described herein, as measured according to routine techniques in the diagnostic art. Relevant physiological or cellular responses (in vivo or in vitro) will be apparent to persons skilled in the art and may include reductions in the symptoms or pathology of a disease associated with mutations to SOD1, such as ALS. A "decrease" in a response may be statistically significant as compared to the response produced by no AON or a control composition, and may include a 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% decrease, including all integers in between.

### Modified AONs

In some embodiments, the AONs have the chemical composition of a naturally occurring nucleic acid molecule, i.e., the AONs do not include a modified or substituted base, sugar, or inter-subunit linkage.

In a preferred embodiment, the AONs of the present invention are non-naturally occurring nucleic acid molecules, or "oligonucleotide analogs". For example, non-naturally occurring nucleic acids can include one or more non-natural base, sugar, and/or inter-subunit linkage, e.g., a base, sugar, and/or linkage that has been modified or substituted with respect to that found in a naturally occurring nucleic acid molecule. Exemplary modifications are described below. In some embodiments, non-naturally occurring nucleic acids include more than one type of modification, e.g. sugar and base modifications, sugar and linkage modifications, base and linkage modifications, or base, sugar, and linkage modifications. For example, in some embodiments, the AONs contain a non-natural (e.g. modified or substituted) base. In some embodiments, the AONs contain a non-natural (e.g. modified or substituted) sugar. In some embodiments, the AONs contain a non-natural (e.g. modified or substituted) inter-subunit linkage. In some embodiments, the AONs contain more than one type of modification or substitution, e.g. a non-natural base and/or a non- natural sugar, and/or a non-natural inter-subunit linkage.

Thus, included are non-naturally-occurring AONs having (i) a modified backbone structure, e.g., a backbone other than the standard phosphodiester linkage found in naturally-occurring oligo- and polynucleotides, and/or (ii) modified sugar moieties, e.g., morpholino moieties rather than ribose or deoxyribose moieties. Oligonucleotide analogs support bases capable of hydrogen bonding by Watson-Crick base pairing to standard polynucleotide bases, where the analog backbone presents the bases in a manner to permit such hydrogen bonding in a sequence-specific fashion between the oligonucleotide analog molecule and bases in a standard polynucleotide (e.g., single-stranded RNA or single-stranded DNA). Preferred analogs are those having a substantially uncharged, phosphorus containing backbone.

One method for producing AONs is the methylation of the 2' hydroxyribose position and the incorporation of a phosphorothioate backbone produces molecules that superficially resemble RNA but that are much more resistant to nuclease degradation, although persons skilled in the art of the invention will be aware of other forms of suitable backbones that may be useable in the objectives of the invention.

To avoid degradation of pre-RNA during duplex formation with the antisense oligomers, the AONs used in the method may be adapted to minimise or prevent cleavage by endogenous Rnase H. Antisense molecules that do not activate Rnase H can be made in accordance with known techniques (see, e.g., U.S. Pat. No. 5,149,797). Such antisense molecules, which may be deoxyribonucleotide or ribonucleotide sequences, simply contain any structural modification which sterically hinders or prevents binding of Rnase H to a duplex molecule containing the oligonucleotide as one member thereof, which structural modification does not substantially hinder or disrupt duplex formation. Because the portions of the oligonucleotide involved in duplex formation are substantially different from those portions involved in Rnase H binding thereto, numerous antisense molecules that do not activate Rnase H are available. This property is highly preferred, as the treatment of the RNA with the unmethylated oligomers, either intracellular or in crude extracts that contain Rnase H, leads to degradation of the pre-mRNA:AON duplexes. Any form of modified AONs that is capable of by-passing or not inducing such degradation may be used in the present method. The nuclease resistance may be achieved by modifying the AONs of the invention so that it comprises partially unsaturated aliphatic hydrocarbon chain and one or more polar or charged groups including carboxylic acid groups, ester groups, and alcohol groups.

An example of AONs which when duplexed with RNA are not cleaved by cellular Rnase H is 2'-O-methyl derivatives. Such 2'-O-methyl-oligoribonucleotides are stable in a cellular environment and in animal tissues, and their duplexes with RNA have higher Tm values than their ribo- or deoxyribo- counterparts. Alternatively, the nuclease resistant AONs of the invention may have at least one of the last 3'-terminus nucleotides fluoridated. Still alternatively, the nuclease resistant AONs of the invention have phosphorothioate bonds linking between at least two of the last 3-terminus nucleotide bases, preferably having phosphorothioate bonds linking between the last four 3'-terminal nucleotide bases.

Decreased RNA cleavage may also be achieved with alternative oligonucleotide chemistry (see, e.g., U.S. Pat. No. 5,149,797). For example, the AON may be chosen from the list comprising: phosphoramidate or phosphorodiamidate morpholino oligomer (PMO); PMO-X; PPMO; peptide nucleic acid (PNA); a locked nucleic acid (LNA) and derivatives including alpha-L-LNA, 2'-amino LNA, 4'-methyl LNA and 4'-O-methyl LNA; ethylene bridged nucleic acids (ENA) and their derivatives; phosphorothioate oligomer; tricyclo-DNA oligomer (tcDNA); tricyclophosphorothioate oligomer; 2'O-Methyl-modified oligomer (2'-Ome); 2'-O-methoxy ethyl (2'-MOE); 2'-fluoro, 2'-fluroarabino (FANA); unlocked nucleic acid (UNA); hexitol nucleic acid (HNA); cyclohexenyl nucleic acid (CeNA); 2'-amino (2'-NH2); 2'-O-ethyleneamine or any combination of the foregoing as mixmers or as gapmers.

In an aspect, the modified AON of the invention can be conjugated to a peptide. Preferably, the AON is a PPMO, i.e. a PMO oligonucleotide chemically conjugated to a peptide moiety via amide, maleimide or click chemistry (preferably using copper-free click chemistry for example via cyclooctyne linkage) and includes suitable linkers, such as cleavable or pH-sensitive linkers. The peptide moiety may be linked via either the 3' or the 5' terminus. Most preferably, the peptide moiety is a peptide that is capable of improving the capacity of the AON to penetrate the cell and reach the nucleus. For example, the peptide moiety can be an arginine-rich peptide, cationic peptide and/or a peptide selected from a library of peptides derived from genomes of biodiverse microorganisms (Hoffman et al., Sci Rep, 8, 1, 12538). The peptides may, or may not contain non-natural amino acids and/or chemically modified amino acids.

Cell penetrating peptides have been added to phosphorodiamidate morpholino oligomers to enhance cellular uptake and nuclear localization. Different cell penetrating peptides have been shown to influence efficiency of uptake and target tissue specificity, as shown in Jearawiriyapaisarn et al. (2008), Mol. Ther., 16(9), 1624-1629. The terms "cell penetrating peptide" and "CPP" are used interchangeably and refer to cationic cell penetrating peptides, also called transport peptides, carrier peptides, or peptide transduction domains. The peptides, as shown herein, have the capability of inducing cell penetration within 100% of cells of a given cell culture population and allow macromolecular translocation within multiple tissues in vivo upon systemic administration. The peptides are also capable of enhancing cellular uptake after localized delivery to a tissue or organ.

To further improve the delivery efficacy, the abovementioned modified nucleotides are often conjugated with fatty acids / lipid / cholesterol / amino acids / carbohydrates / polysaccharides / nanoparticles etc. to the sugar or nucleobase moieties. These conjugated nucleotide derivatives can also be used to construct AONs to induce exon skipping. Antisense oligomer-induced exon skipping of the human *SOD1* gene transcripts can use oligoribonucleotides, PNAs, 2Ome or MOE modified bases on a phosphorothioate backbone. Although 2OMeAONs are used for oligo design, due to their efficient uptake in vitro when delivered as cationic lipoplexes, these compounds are susceptible to nuclease degradation and are not considered ideal for in vivo or clinical applications. When alternative chemistries are used to generate the AONs of the present invention, the uracil (U) of the sequences provided herein may be replaced by a thymine (T).

For example, the AONs of the present invention have sequences SEQ ID NO: 22 - SEQ ID NO: 42 as listed in Table 2:

**Table 2**

| **SEQ ID NO** | **Co-ordinates** | **Sequence 5'→3'** |
|---|---|---|
| 22 | *SOD1* H2A (-12+13) | GTCCATTACTTTCCTTTAAGAAAAG |
| 23 | *SOD1* H2A (+18+43) | GTCCTTTAATGCTTCCCCACACCTT |
| 24 | *SOD1* H2A (+35+57) | CAGGCTTCAGTCAGTCCTTTAA |
| 25 | *SOD1* H2A (+54+79) | ACTCATGAACATGGAATCCATGCAGG |
| 26 | *SOD1* H2D (+13-08) | ACACCCACCTGCTGTATTATC |
| 27 | *SOD1* H2D (+16-09) | AACACCCACCTGCTGTATTATCTCC |
| 28 | *SOD1* H3A (+05+27) | TAAAGTGAGGACCTGCACTGGTA |
| 29 | *SOD1* H3A (+37+62) | ATCCTTTGGCCCACCGTGTTTTCTG |
| 30 | *SOD1* H3A (+42+63) | CATCCTTTGGCCCACCGTGTT |
| 31 | *SOD1* H3D (+20-05) | GTTACCTCTCTTCATCCTTTGGCCC |
| 32 | *SOD1* H4A (-16+09) | CCAACATGCCTAATAATGAAAAAGC |
| 33 | *SOD1* H4A (+15+37) | TTTGTCAGCAGTCACATTGCCCA |
| 34 | *SOD1* H4A (+38+62) | CAATAGACACATCGGCCACACCATC |
| 35 | *SOD1* H4A (+79+101) | TGATGCAATGGTCTCCTGAGAGT |
| 36 | *SOD1* H4D (+06-19) | TCCTTTTATGAAAACTTACCACCAG |
| 37 | *SOD1* H1A(+182+206) | TGATGATGCCCTGCACTGGGCCGTC |
| 38 | *SOD1* H1D(+20-05) | CTTGCCTTCTGCTCGAAATTGATGA |
| 39 | *SOD1* H2D(+21-04) | CCACCTGCTGTATTATCTCCAAACT |
| 40 | *SOD1* H3A(+32+57) | TTGGCCCACCGTGTTTTCTGGATAG |
| 41 | *SOD1* H4A(-05+24) | ATTAGGCATGTTGGAGACTTGGGCA |
| 42 | *SOD1* H4A(+28+52) | TGCTGACAAAGATGGTGTGGCCGAT |
| 43 | | AACACCCAC |
| 44 | | CUGCUGUAUUAUCUCC |
| 45 | | CTGCTGTATTATCTCC |
| 46 | | CGACGAAGGCCGTGTGCGTG |
| 47 | | AGTTAAGGGGCCTCAGACTACA |

| | | |
|---|---|---|
| *Reverse complement sequence shown 5-3'. The reference point (0) set at first base of the 5' and 3' splice sites; hence* "+" *refers to nucleotides binding within the exon and* "-" *indicates nucleotides binding within the intron.* | | |

For example, such antisense molecules may be oligonucleotides wherein at least one, or all, of the inter-nucleotide bridging phosphate residues are modified phosphates, such as methyl phosphonates, methyl phosphorothioates, phosphoromorpholidates, phosphoropiperazidates and phosphor amidates. For example, every other one of the internucleotide bridging phosphate residues may be modified as described. In another non-limiting example, such antisense molecules are molecules wherein at least one, or all, of the nucleotides contain a 2' lower alkyl moiety (e.g., Ci-C4, linear or branched, saturated or unsaturated alkyl, such as methyl, ethyl, ethenyl, propyl, 1-propenyl, 2-propenyl, and isopropyl). For example, every other one of the nucleotides may be modified as described.

Specific examples of AONs useful in this invention include oligonucleotides containing modified backbones or non-natural intersubunit linkages.

Oligonucleotides having modified backbones include those that retain a phosphorus atom in the backbone and those that do not have a phosphorus atom in the backbone. Modified oligonucleotides that do not have a phosphorus atom in their inter-nucleoside backbone can also be considered to be oligonucleosides.

In other antisense molecules, both the sugar and the inter-nucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleo-bases are retained and are bound directly or indirectly to aza nitrogen atoms of the amide portion of the backbone.

Modified oligonucleotides may also contain one or more substituted sugar moieties. Oligonucleotides may also include nucleobase (often referred to in the art simply as "base") modifications or substitutions. Oligonucleotides containing a modified or substituted base include oligonucleotides in which one or more purine or pyrimidine bases most commonly found in nucleic acids are replaced with less common or non-natural bases.

Purine bases comprise a pyrimidine ring fused to an imidazole ring; adenine and guanine are the two purine nucleobases most commonly found in nucleic acids. These may be substituted with other naturally-occurring purines, including but not limited to N₆-methyladenine, N₂-methylguanine, hypoxanthine, and 7-methylguanine.

Pyrimidine bases comprise a six-membered pyrimidine ring; cytosine, uracil, and thymine are the pyrimidine bases most commonly found in nucleic acids. These may be substituted with other naturally-occurring pyrimidines, including but not limited to 5-methylcytosine, 5-hydroxymethylcytosine, pseudouracil, and 4-thiouracil. In one embodiment, the oligonucleotides described herein contain thymine bases in place of uracil.

Other modified or substituted bases include, but are not limited to, 2,6-diaminopurine, orotic acid, agmatidine, lysidine, 2-thiopyrimidine (e.g. 2-thiouracil, 2-thiothymine), G-clamp and its derivatives, 5-substituted pyrimidine (e.g. 5-halouracil, 5-propynyluracil, 5-propynylcytosine, 5- aminomethyluracil, 5-hydroxymethyluracil, 5-aminomethylcytosine, 5-hydroxymethylcytosine, Super T), 7-deazaguanine, 7-deazaadenine, 7-aza-2,6-diaminopurine, 8-aza-7-deazaguanine, 8-aza- 7-deazaadenine, 8-aza-7-deaza-2,6-diaminopurine, Super G, Super A, and N4-ethylcytosine, or derivatives thereof; N₂-cyclopentylguanine (cPent-G), N₂-cyclopentyl-2-aminopurine (cPent-AP), and N₂-propyl-2-aminopurine (Pr-AP), pseudouracil or derivatives thereof; and degenerate or universal bases, like 2,6-difluorotoluene or absent bases like abasic sites (e.g. 1-deoxyribose, 1,2-dideoxyribose, 1-deoxy-2-O-methylribose; or pyrrolidine derivatives in which the ring oxygen has been replaced with nitrogen (azaribose)). Examples of derivatives of Super A, Super G and Super T can be found in U.S. Patent 6,683, 173 (Epoch Biosciences). cPent-G, cPent-AP and Pr-AP were shown to reduce immunostimulatory effects when incorporated in siRNA (Peacock H. et al. J. Am. Chem. Soc. 2011, 133, 9200). Pseudouracil is a naturally occurring isomerized version of uracil, with a C-glycoside rather than the regular N- glycoside as in uridine. Pseudouridine -containing synthetic mRNA may have an improved safety profile compared to uridine-containing mPvNA (see WO 2009127230).

Certain modified or substituted nucleo-bases are particularly useful for increasing the binding affinity of the AONs of the invention. These include 5-substituted pyrimidines, 6-azapyrimidines and N-2, N-6 and 0-6 substituted purines, including 2-aminopropyladenine, 5-propynyluracil and 5-propynylcytosine. 5-methylcytosine substitutions have been shown to increase nucleic acid duplex stability by 0.6-1.2°C and are presently preferred base substitutions, even more particularly when combined with 2'-0-methoxyethyl sugar modifications.

In some embodiments, modified or substituted nucleo-bases are useful for facilitating purification of AONs. For example, in certain embodiments, AONs may contain three or more (e.g., 3, 4, 5, 6 or more) consecutive guanine bases. In certain AONs, a string of three or more consecutive guanine bases can result in aggregation of the oligonucleotides, complicating purification. In such AONs, one or more of the consecutive guanines can be substituted with inosine. The substitution of inosine for one or more guanines in a string of three or more consecutive guanine bases can reduce aggregation of the AON, thereby facilitating purification.

In one embodiment, another modification of the AONs involves chemically linking to the oligonucleotide one or more moieties or conjugates that enhance the activity, cellular distribution or cellular uptake of the oligonucleotide. Such moieties include but are not limited to lipid moieties such as a cholesterol moiety, cholic acid, a thioether, e.g., hexyl-5-tritylthiol, a thiocholesterol, an aliphatic chain, e.g., dodecandiol or undecyl residues, a phospholipid, e.g., di-hexadecyl-rac-glycerol or triethylammonium 1 ,2-di-O-hexadecyl-rac-glycero-3-H-phosphonate, a polyamine or a polyethylene glycol chain, or adamantane acetic acid, a palmityl moiety, or an octadecylamine or hexylamino-carbonyl-oxycholesterol moiety.

It is not necessary for all positions in a given compound to be uniformly modified, and in fact more than one of the aforementioned modifications may be incorporated in a single compound or even at a single nucleoside within an oligonucleotide. The present invention also includes AONs that are chimeric compounds. "Chimeric" antisense compounds or "chimeras," in the context of this invention, are antisense molecules, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the increased resistance to nuclease degradation, increased cellular uptake, and an additional region for increased binding affinity for the target nucleic acid.

The antisense molecules used in accordance with this invention may be conveniently and routinely made through the well-known technique of solid phase synthesis. Equipment for such synthesis is sold by several vendors including, for example, Applied Biosystems (Foster City, Calif.). One method for synthesising oligonucleotides on a modified solid support is described in U.S. Pat. No. 4,458,066.

In another non-limiting example, such AONs are molecules wherein at least one, or all, of the nucleotides contain a 2' lower alkyl moiety (such as, for example, C₁-C₄, linear or branched, saturated or unsaturated alkyl, such as methyl, ethyl, ethenyl, propyl, 1-propenyl, 2-propenyl, and isopropyl). For example, every other one of the nucleotides may be modified as described.

While the AONs described above are a preferred form of the AONs of the present invention, the present invention includes other oligomeric antisense molecules, including but not limited to oligomer mimetics such as are described below.

Another preferred chemistry is the phosphorodiamidate morpholino oligomer (PMO) oligomeric compounds, which are not degraded by any known nuclease or protease. These compounds are uncharged, do not activate RNase H activity when bound to a RNA strand and have been shown to exert sustained cleavage factor binding modulation after in vivo administration (Summerton and Weller, Antisense Nucleic Acid Drug Development, 7, 187-197).

Modified oligomers may also contain one or more substituted sugar moieties. Oligomers may also include nucleobase (often referred to in the art simply as "base") modifications or substitutions. Certain nucleobases are particularly useful for increasing the binding affinity of the oligomeric compounds of the invention. These include 5-substituted pyrimidines, 6-azapyrimidines, and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2°C, even more particularly when combined with 2'-O-methoxyethyl sugar modifications. In one embodiment, at least one pyrimidine base of the oligonucleotide comprises a 5-substituted pyrimidine base, wherein the pyrimidine base is selected from the group consisting of cytosine, thymine and uracil. In one embodiment, the 5- substituted pyrimidine base is 5-methylcytosine. In another embodiment, at least one purine base of the oligonucleotide comprises an N-2, N-6 substituted purine base. In one embodiment, the N- 2, N-6 substituted purine base is 2, 6-diaminopurine.

In one embodiment, the AON includes one or more 5-methylcytosine substitutions alone or in combination with another modification, such as 2'-0-methoxyethyl sugar modifications. In yet another embodiment, the AON includes one or more 2, 6-diaminopurine substitutions alone or in combination with another modification.

In some embodiments, the AON is chemically linked to one or more moieties, such as a polyethylene glycol moiety, or conjugates, such as an arginine-rich cell penetrating peptide that enhance the activity, cellular distribution, or cellular uptake of the AON. In one exemplary embodiment, the arginine-rich polypeptide is covalently coupled at its N-terminal or C-terminal residue to the 3' or 5' end of the antisense compound. Also, in an exemplary embodiment, the antisense compound is composed of morpholino subunits and phosphorus-containing inter-subunit linkages joining a morpholino nitrogen of one subunit to a 5' exocyclic carbon of an adjacent subunit.

In another aspect, the invention provides expression vectors that incorporate the AONs described above, e.g., the AONs of SEQ ID NOs: 1-42. In some embodiments, the expression vector is a modified retrovirus or non-retroviral vector, such as an adeno-associated viral vector.

### Assays for measuring activity of AONs

The activity of AONs and variants thereof can be assayed according to routine techniques in the art. For example, isoform forms and expression levels of surveyed RNAs and proteins may be assessed by any of a wide variety of well-known methods for detecting isoforms and/or expression of a transcribed nucleic acid or protein. Non-limiting examples of such methods include RT-PCR of isoforms of RNA followed by size separation of PCR products, nucleic acid hybridization methods e.g., Northern blots and/or use of nucleic acid arrays; fluorescent in situ hybridization to detect RNA transcripts inside cells; nucleic acid amplification methods; immunological methods for detection of proteins; protein purification methods; and protein function or activity assays.

RNA expression levels can be assessed by preparing RNA/cDNA (i.e., a transcribed polynucleotide) from a cell, tissue or organism, and by hybridizing the RNA/cDNA with a reference polynucleotide, which is a complement of the assayed nucleic acid, or a fragment thereof. cDNA can, optionally, be amplified using any of a variety of polymerase chain reaction or in vitro transcription methods prior to hybridization with the complementary polynucleotide; preferably, it is not amplified. Expression of one or more transcripts can also be detected using quantitative PCR to assess the level of expression of the transcript(s).

### Methods of manufacturing AONs

The AONs used in accordance with this invention may be conveniently made through the well-known technique of solid phase synthesis. Equipment for such synthesis is sold by several vendors including, for example, Applied Biosystems (Foster City, Calif.). One method for synthesising oligomers on a modified solid support is described in U.S. Pat. No. 4,458,066.

Any other means for such synthesis known in the art may additionally or alternatively be employed. It is well known to use similar techniques to prepare oligomers such as the phosphorothioates and alkylated derivatives. In one such automated embodiment, diethyl-phosphoramidites are used as starting materials and may be synthesized as described by Beaucage, et al., (1981) Tetrahedron Letters, 22:1859-1862.

The AONs of the invention are synthesised *in vitro* and do not include antisense compositions of biological origin, or genetic vector constructs designed to direct the *in vivo* synthesis of antisense oligomers. The molecules of the invention may also be mixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption.

### Vectors

Also included are vector delivery systems that are capable of expressing the oligomeric, SOD1-targeting sequences of the present invention, such as vectors that express a polynucleotide sequence comprising any one or more of SEQ ID NOs: 1-42, as described herein.

By "vector" or "nucleic acid construct" is meant a polynucleotide molecule, preferably a DNA molecule derived, for example, from a plasmid, bacteriophage, yeast or virus, into which a polynucleotide can be inserted or cloned. A vector preferably contains one or more unique restriction sites and can be capable of autonomous replication in a defined host cell including a target cell or tissue or a progenitor cell or tissue thereof or able to be integrated with the genome of the defined host such that the cloned sequence is reproducible.

Accordingly, the vector can be an autonomously replicating vector, i.e., a vector that exists as an extra-chromosomal entity, the replication of which is independent of chromosomal replication, e.g., a linear or closed circular plasmid, an extra-chromosomal element, a mini-chromosome, or an artificial chromosome. The vector can contain any means for assuring self-replication. Alternatively, the vector can be one which, when introduced into the host cell, is integrated into the genome and replicated together with the chromosome(s) into which it has been integrated.

### C. Method of Treatment

The AONs of the present invention also can be used as a prophylactic or therapeutic, which may be utilised for the purpose of treatment of a disease. Accordingly, in one embodiment the present invention provides AONs that bind to a selected target in the SOD1 RNA to reduce expression of *SOD1* as described herein, in a therapeutically effective amount, admixed with a pharmaceutically acceptable carrier, diluent, or excipient.

An "effective amount" or "therapeutically effective amount" refers to an amount of therapeutic compound, such as an antisense oligomer, administered to a mammalian subject, either as a single dose or as part of a series of doses, which is effective to produce a desired therapeutic effect.

The invention therefore provides a pharmaceutical, prophylactic, or therapeutic composition to treat, prevent or ameliorate the effects of a disease associated with mutations or misfolding to SOD1, the composition comprising:
a) one or more AONs as described herein, and
b) one or more pharmaceutically acceptable carriers and/or diluents.

Preferably, the disease associated with misfolding or mutations to *SOD1* is ALS.

However, patients suffering from ALS that do not necessarily exhibit mutations or misfolding in SOD1 may also be treated by the AONs of this invention. Therefore in another aspect, the invention provides a pharmaceutical, prophylactic, or therapeutic composition to treat, prevent or ameliorate the effects of ALS, the composition comprising:
a) one or more AONs as described herein, and
b) one or more pharmaceutically acceptable carriers and/or diluents.

There is also provided a method for treating, preventing or ameliorating the effects of a disease associated with mutations in SOD1, the method comprising the step of: administering to the subject an effective amount of one or more AONs or pharmaceutical composition comprising one or more AONs as described herein.

The invention provides a method for treating, preventing or ameliorating the effects of a disease associated with misfolding in SOD1, the method comprising the step of: administering to the subject an effective amount of one or more AONs or pharmaceutical composition comprising one or more AONs as described herein.

There is also provided a method for treating, preventing or ameliorating the effects of ALS, the method comprising the step of: administering to the subject an effective amount of one or more AONs or pharmaceutical composition comprising one or more AONs as described herein.

In a further aspect, genetic or other biomarkers can be used to identify ALS patients most likely to respond well to SOD1 suppression via the AONs of the invention. Genetic structural variations associated with ALS disease risk have been identified within ALS genes and surrounding gene regions. These variations can be used as genetic biomarkers to identify patients likely to respond to the methods of this invention. Non-genetic biomarkers can also be used to identify patients likely to respond to the methods of this invention.

The invention provides a method for treating, preventing or ameliorating the effects of ALS, in subjects identified by a biomarker, the method comprising the step of:
a) testing a subject for the presence of a biomarker associated with ALS patients likely to respond to SOD1 suppression; and
b) if the subject is found to express the biomarker, administering to the subject an effective amount of one or more AONs or pharmaceutical composition comprising one or more AONs as described herein.

Preferably, the biomarker is a genetic structural variant within the SOD1 gene region, for example, the structural variant within *SCAF4,* or a genetic structural variant within a SOD1 related/interacting gene region, for example, within *SQSTM1.* The detection of misfolded SOD1 within the CSF may also be used as a biomarker. The biomarkers can be identified in patients through assays known in the art.

There is also provided herein the use of purified and isolated AONs as described herein, to treat, prevent or ameliorate the effects of a disease associated with mutations or misfolding in SOD1.

There is also provided herein the use of purified and isolated AONs as described herein, to treat, prevent or ameliorate the effects of ALS.

Preferably, the AON used in the present invention is chosen from the list of AONs provided in Tables 1 or 2 or more preferably is selected from SEQ ID NO: 6, 8, 13 27, 29 or 34. By way of example the AON is SEQ ID NO: 8 or 29.

The composition may comprise about 1 nM to 1000 µM of each of the desired antisense oligomer(s) of the invention. Preferably, the composition may comprise about 1 µM to 500 µM, 10 µM to 500 µM, 50 µM to 750 µM, 10 µM to 500 µM, 1 µM to 100 µM, 1 µM to 50 µM, preferably between 25 µM and 100 µM of each of the antisense oligomer(s) of the invention. The composition may also preferably comprise about 1 nM to 500 nM, 10 nM to 500 nM, 50 nM to 750 nM, 10 nM to 500 nM, 1 nM to 100 nM, 1 nM to 50 nM, most preferably between 50 nM and 100 nM of each of the antisense oligomer(s) of the invention.

The composition may comprise about 1nM, 2nM, 3nM, 4nM, 5nM, 6nM, 7nM, 8nM, 9nM, 10nM, 20nM, 50nM, 75nM, 100nM, 150nM, 200nM, 250nM, 300nM, 350nM, 400nM, 450nM, 500nM, 550nM, 600nM, 650nM, 700nM, 750nM, 800nM, 850nM, 900nM, 950nM or 1000nM of each of the desired antisense oligomer(s) of the invention.

The present invention further provides one or more AONs adapted to aid in the prophylactic or therapeutic treatment, prevention or amelioration of symptoms of a disease or pathology associated with mutations to *SOD1* in a form suitable for delivery to a subject.

The phrase "pharmaceutically acceptable" refers to molecular entities and compositions that are physiologically tolerable and do not typically produce an allergic or similarly untoward reaction, such as gastric upset and the like, when administered to a subject. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or saline solutions and aqueous dextrose and glycerol solutions are preferably employed as carriers, particularly for injectable solutions. Suitable pharmaceutical carriers are described in Martin, Remington's Pharmaceutical Sciences, 18th Ed., Mack Publishing Co., Easton, PA, (1990).

### D. Manufacture of a Medicament

In one embodiment, the present invention provides the use of AONs that bind to a selected target in the SOD1 RNA for the manufacture of a medicament to treat, prevent or ameliorate the effects of ALS. There is therefore provided the use of one or more AONs described herein for the manufacture of a medicament to treat, prevent or ameliorate the effects of ALS.

There is also provided the use of one or more AONs described herein for the manufacture of a medicament to treat, prevent or ameliorate the effects of a disease associated with mutations in SOD1.

There is provided the use of one or more AONs described herein for the manufacture of a medicament to treat, prevent or ameliorate the effects of a disease associated with misfolding in SOD1.

The invention provides the use of purified and isolated antisense oligonucleotides according as described herein, for the manufacture of a medicament to treat, prevent or ameliorate the effects of a disease associated with mutations in *SOD1.*

There is also provided use of purified and isolated antisense oligonucleotides according as described herein, for the manufacture of a medicament to treat, prevent or ameliorate the effects of a disease associated with misfolding in *SOD1.*

The invention also provides the use of purified and isolated antisense oligonucleotides according as described herein, for the manufacture of a medicament to treat, prevent or ameliorate the effects of ALS.

There is also provided the use of one or more AONs described herein for the manufacture of a medicament to treat, prevent or ameliorate the effects of ALS in subjects expressing a biomarker associated with ALS patients likely to respond to SOD1 suppression.

Preferably, the biomarker is a genetic structural variant within the SOD1 gene region, for example, the structural variant within *SCAF4,* or a genetic structural variant within a SOD1 related/interacting gene region, for example, within *SQSTM1.* The detection of misfolded SOD1 within the CSF may also be used as a biomarker. The biomarkers can be identified in patients through assays known in the art.

Preferably, the AON used for the manufacture of a medicament is chosen from the list of AONs provided in Tables 1 or 2 or more preferably is selected from SEQ ID NO: 6, 8, 13 27, 29 or 34. By way of example the AON is SEQ ID NO: 8 or 29.

### E. Pharmaceutical Compositions

In a form of the invention there are provided pharmaceutical compositions comprising therapeutically effective amounts of one or more AONs of the invention together with pharmaceutically acceptable diluents, preservatives, solubilizers, emulsifiers, adjuvants, and/or carriers. Such compositions include diluents of various buffer content (e.g. Tris-HCl, acetate, phosphate), pH and ionic strength and additives such as detergents and solubilizing agents (e.g. Tween 80, Polysorbate 80), anti-oxidants (e.g., ascorbic acid, sodium metabisulfite), preservatives (e.g. Thimersol, benzyl alcohol) and bulking substances (e.g., lactose, mannitol). The material may be incorporated into particulate preparations of polymeric compounds such as polylactic acid, polyglycolic acid, etc. or into liposomes. Hyalluronic acid may also be used. Such compositions may influence the physical state, stability, rate of in vivo release, and rate of in vivo clearance of the present proteins and derivatives. See, for example, Martin, Remington's Pharmaceutical Sciences, 18th Ed. (1990, Mack Publishing Co., Easton, PA 18042) pages 1435-1712 that are herein incorporated by reference. The compositions may be prepared in liquid form, or may be in dried powder, such as a lyophilised form.

It will be appreciated that pharmaceutical compositions provided according to the present invention may be administered by any means known in the art. Preferably, the pharmaceutical compositions for administration are administered by injection, orally, topically or by the pulmonary or nasal route. The AONs are more preferably delivered by intravenous, intra-arterial, intraperitoneal, intramuscular or subcutaneous routes of administration. The appropriate route may be determined by one of skill in the art, as appropriate to the condition of the subject under treatment. Vascular or extravascular circulation, the blood or lymph system, and the cerebrospinal fluid are some non-limiting sites where the AON may be introduced. Direct CNS delivery may be employed, for instance, intracerebro-ventricular or intrathecal administration may be used as routes of administration.

Formulations for topical administration include those in which the oligomers of the disclosure are in admixture with a topical delivery agent such as lipids, liposomes, fatty acids, fatty acid esters, steroids, chelating agents and surfactants. Lipids and liposomes include neutral (e.g. dioleoylphosphatidyl DOPE ethanolamine, dimyristoylphosphatidyl choline DMPC, distearolyphosphatidyl choline) negative (e.g. dimyristoylphosphatidyl glycerol DMPG) and cationic (e.g. dioleoyltetramethylaminopropyl DOTAP and dioleoylphosphatidyl ethanolamine DOTMA). For topical or other administration, oligomers of the disclosure may be encapsulated within liposomes or may form complexes thereto, in particular to cationic liposomes. Alternatively, oligomers may be complexed to lipids, in particular to cationic lipids. Fatty acids and esters, pharmaceutically acceptable salts thereof, and their uses are further described in U.S. Pat. No. 6,287,860 and/or U.S. patent application Ser. No. 09/315,298 filed on May 20, 1999.

In certain embodiments, the AONs of the disclosure can be delivered by transdermal methods (e.g., via incorporation of the AONs into, e.g., emulsions, with such AONs optionally packaged into liposomes). Such transdermal and emulsion/liposome-mediated methods of delivery are described for delivery of AONs in the art, e.g., in U.S. Pat. No. 6,965,025.

The AONs described herein may also be delivered via an implantable device. Design of such a device is an art-recognized process, with, e.g., synthetic implant design described in, e.g., U.S. Pat. No. 6,969,400.

Compositions and formulations for oral administration include powders or granules, microparticulates, nanoparticulates, suspensions or solutions in water or nonaqueous media, capsules, gel capsules, sachets, tablets or minitablets. Thickeners, flavoring agents, diluents, emulsifiers, dispersing aids or binders may be desirable. Oral formulations are those in which oligomers of the disclosure are administered in conjunction with one or more penetration enhancers surfactants and chelators. Surfactants include fatty acids and/or esters or salts thereof, bile acids and/or salts thereof. Bile acids/salts and fatty acids and their uses are further described in U.S. Pat. No. 6,287,860. In some embodiments, the present disclosure provides combinations of penetration enhancers, for example, fatty acids/salts in combination with bile acids/salts. An exemplary combination is the sodium salt of lauric acid, capric acid and UDCA. Further penetration enhancers include polyoxyethylene-9-lauryl ether, polyoxyethylene-20-cetyl ether. Oligomers of the disclosure may be delivered orally, in granular form including sprayed dried particles, or complexed to form micro or nanoparticles. Oligomer complexing agents and their uses are further described in U.S. Pat. No. 6,287,860. Oral formulations for oligomers and their preparation are described in detail in U.S. 6,887,906, 09/315,298 filed May 20, 1999 and/or US20030027780.

Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

The delivery of a therapeutically useful amount of AONs may be achieved by methods previously published. For example, intracellular delivery of the AON may be via a composition comprising an admixture of the AON and an effective amount of a block copolymer. An example of this method is described in US patent application US20040248833. Other methods of delivery of AONs to the nucleus are described in Mann CJ et al. (2001) Proc, Natl. Acad. Science, 98(1) 42-47, and in Gebski et al. (2003) Human Molecular Genetics, 12(15): 1801-1811. A method for introducing a nucleic acid molecule into a cell by way of an expression vector either as naked DNA or complexed to lipid carriers, is described in US 6,806,084.

In certain embodiments, the AONs of the invention and therapeutic compositions comprising the same can be delivered by transdermal methods (e.g., via incorporation of the AONs into, e.g., emulsions, with such AONs optionally packaged into liposomes). Such transdermal and emulsion/liposome-mediated methods of delivery are described for delivery of AONs in the art, e.g., in U.S. Pat. No. 6,965,025.

It may be desirable to deliver the AON in a colloidal dispersion system. Colloidal dispersion systems include macromolecule complexes, nanocapsules, microspheres, beads, and lipid-based systems including oil-in-water emulsions, micelles, mixed micelles, and liposomes or liposome formulations. These colloidal dispersion systems can be used in the manufacture of therapeutic pharmaceutical compositions.

Liposomes are artificial membrane vesicles, which are useful as delivery vehicles in vitro and in vivo. These formulations may have net cationic, anionic, or neutral charge characteristics and have useful characteristics for in vitro, in vivo and ex vivo delivery methods. It has been shown that large unilamellar vesicles can encapsulate a substantial percentage of an aqueous buffer containing large macromolecules. RNA and DNA can be encapsulated within the aqueous interior and be delivered to cells in a biologically active form (Fraley, et al., 1981, Trends Biochem. Sci., 6, 77).

In order for a liposome to be an efficient gene transfer vehicle, the following characteristics should be present: (1) encapsulation of the AON of interest at high efficiency while not compromising their biological activity; (2) preferential and substantial binding to a target cell in comparison to non-target cells; (3) delivery of the aqueous contents of the vesicle to the target cell cytoplasm at high efficiency; and (4) accurate and effective expression of genetic information (Mannino, et al., 1988 Biotechniques, 6, 682). The composition of the liposome is usually a combination of phospholipids, particularly high phase-transition-temperature phospholipids, usually in combination with steroids, especially cholesterol. Other phospholipids or other lipids may also be used. The physical characteristics of liposomes depend on pH, ionic strength, and the presence of divalent cations. Cationic liposomes are positively charged liposomes which are believed to interact with negatively charged DNA molecules to form a stable complex. Liposomes that are pH-sensitive or negatively-charged are believed to entrap DNA rather than complex with it. Both cationic and noncationic liposomes have been used to deliver DNA to cells.

Liposomes also include "sterically stabilized" liposomes, a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome comprises one or more glycolipids or is derivatized with one or more hydrophilic polymers, such as a polyethylene glycol (PEG) moiety. Liposomes and their uses are further described in U.S. 6,287,860.

The AONs described herein may also be delivered via an implantable device. Design of such a device is an art-recognized process, with, e.g., synthetic implant design described in, e.g., U.S. Pat. No. 6,969,400, the contents of which are incorporated in their entirety by reference herein.

AONs can be introduced into cells using art-recognized techniques (e.g., transfection, electroporation, fusion, liposomes, colloidal polymeric particles and viral and non-viral vectors as well as other means known in the art). The method of delivery selected will depend at least on the cells to be treated and the location of the cells and will be apparent to the skilled artisan. For instance, localization can be achieved by liposomes with specific markers on the surface to direct the liposome, direct injection into tissue containing target cells, specific receptor-mediated uptake, or the like.

As known in the art, AONs may be delivered using, for example, methods involving liposome-mediated uptake, lipid conjugates, polylysine-mediated uptake, nanoparticle-mediated uptake, and receptor-mediated endocytosis, as well as additional non-endocytic modes of delivery, such as microinjection, permeabilization (e.g., streptolysin-O permeabilization, anionic peptide permeabilization), electroporation, and various non-invasive non-endocytic methods of delivery that are known in the art (refer to Dokka and Rojanasakul, Advanced Drug Delivery Reviews 44, 35-49, incorporated by reference in its entirety).

The AON may also be combined with other pharmaceutically acceptable carriers or diluents to produce a pharmaceutical composition. Suitable carriers and diluents include isotonic saline solutions, for example phosphate-buffered saline. The composition may be formulated for parenteral, intramuscular, intravenous, subcutaneous, intraocular, oral, or transdermal administration.

The routes of administration described are intended only as a guide since a skilled practitioner will be able to readily determine the optimum route of administration and any dosage for any particular animal and condition.

Multiple approaches for introducing functional new genetic material into cells, both in vitro and in vivo have been attempted (Friedmann (1989) Science, 244, 1275-1280). These approaches include integration of the gene to be expressed into modified retroviruses (Friedmann (1989) *supra;* Rosenberg (1991) Cancer Research 51(18), suppl.: 5074S-5079S); integration into non-retrovirus vectors (Rosenfeld, et al. (1992) Cell, 68, 143-155; Rosenfeld, et al. (1991) Science, 252, 431-434); or delivery of a transgene linked to a heterologous promoter-enhancer element via liposomes (Friedmann (1989), *supra;* Brigham, et al. (1989) Am. J. Med. Sci., 298, 278-281; Nabel, et al. (1990) Science, 249, 1285-1288; Hazinski, et al. (1991) Am. J. Resp. Cell Molec. Biol., 4:206-209; and Wang and Huang (1987) Proc. Natl. Acad. Sci. (USA), 84, 7851-7855); coupled to ligand-specific, cation-based transport systems (Wu and Wu (1988) J. Biol. Chem., 263, 14621-14624) or the use of naked DNA, expression vectors (Nabel *et al.* (1990), *supra);* Wolff et al. (1990) Science, 247, 1465-1468). Direct injection of transgenes into tissue produces only localized expression (Rosenfeld (1992) *supra);* Rosenfeld *et al.* (1991) *supra;* Brigham *et al.* (1989) *supra;* Nabel (1990) *supra;* and Hazinski *et al.* (1991) *supra).* The Brigham et al. group ((1989) Am. J. Med. Sci. 298, 278-281 and Clinical Research (1991) 39 (abstract)) have reported *in vivo* transfection only of lungs of mice following either intravenous or intratracheal administration of a DNA liposome complex. An example of a review article of human gene therapy procedures is: Anderson, (1992) Science 256, 808-813; Barteau et al. (2008), Curr Gene Ther., 8(5), 313-23; Mueller et al. (2008). Clin Rev Allergy Immunol., 35(3), 164-78; Li et al. (2006) Gene Ther., 13(18), 1313-9; Simoes et al. (2005) Expert Opin Drug Deliv., 2(2), 237-54.

The AONs of the invention encompass any pharmaceutically acceptable salts, esters, or salts of such esters, or any other compound which, upon administration to an animal including a human, is capable of providing (directly or indirectly) the biologically active metabolite or residue thereof. Accordingly, as an example, the disclosure is also drawn to prodrugs and pharmaceutically acceptable salts of the compounds of the invention, pharmaceutically acceptable salts of such pro-drugs, and other bioequivalents.

The term "pharmaceutically acceptable salts" refers to physiologically and pharmaceutically acceptable salts of the compounds of the invention: i.e. salts that retain the desired biological activity of the parent compound and do not impart undesired toxicological effects thereto. For oligomers, preferred examples of pharmaceutically acceptable salts include but are not limited to (a) salts formed with cations such as sodium, potassium, ammonium, magnesium, calcium, polyamines such as spermine and spermidine, etc.; (b) acid addition salts formed with inorganic acids, for example hydrochloric acid, hydrobromic acid, sulfuric acid, phosphoric acid, nitric acid and the like; (c) salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; and (d) salts formed from elemental anions such as chlorine, bromine, and iodine. The pharmaceutical compositions of the present invention may be administered in a number of ways depending upon whether local or systemic treatment is desired and upon the area to be treated. Administration may be topical (including ophthalmic and mucous membranes, as well as rectal delivery), pulmonary, e.g., by inhalation or insufflation of powders or aerosols (including by nebulizer, intratracheal, intranasal, epidermal and transdermal), oral or parenteral. Parenteral administration includes intravenous, intra-arterial, subcutaneous, intraperitoneal or intramuscular injection or infusion; or intracranial, e.g., intrathecal or intraventricular, administration. Oligomers with at least one 2'-O-methoxyethyl modification are believed to be particularly useful for oral administration. Preferably, the AON is delivered via the subcutaneous or intravenous route.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipients(s). In general, the formulations are prepared by uniformly and intimately bringing into association the active ingredients with liquid carriers or finely divided solid carriers or both, and then, if necessary, shaping the product.

The following Examples are to be construed as merely illustrative and not limitative of the remainder of the disclosure in any way whatsoever. These Examples are included solely for the purposes of exemplifying the present invention. They should not be understood as a restriction on the broad summary, disclosure or description of the invention as set out above. Without further elaboration, it is believed that one skilled in the art can, using the preceding description, utilize the present invention to its fullest extent. In the foregoing and in the following examples, all temperatures are set forth uncorrected in degrees Celsius; and, unless otherwise indicated, all parts and percentages are by weight.

### EXAMPLES

### Examples

### Example 1 - Design of original AONs

Splice-switching AONs were designed to target the 5' and 3' splice sites and exon splice enhancer sites within the exon as predicted by online splice prediction tools.

The sequences for these original AONs are listed in Table 1, as SEQ ID NO 3, 5, 7, 9, 12 and 14. AON nomenclature was based on that described by Mann *et al.* (Mann et al, J Gene Med., 2002 4 (6), 644-654), whereby the species, gene, exon number, acceptor or donor targeting and annealing coordinates are described, where "-" indicates intronic position and "+" specifies exonic location from the splice site, as described herein. Some detailed oligomer annealing coordinates are shown in Table 1. Figure 12 sets out a schematic diagram of the binding location of each of the AONs on *SOD1.*

AONs with 2'OMethyl modifications and a phosphorothioate backbone were ordered from TriLink Biotechnologies, Inc (San Diego, CA, USA). PMOs with a phosphorodiamidate backbone were ordered from Genetools LLC (Philomath, OR, USA).

### Example 2 - AON Screening using Transcript analysis

RT-PCR analysis of the SOD1 transcript was conducted following transfection of 2'OMethyl AONs (50, 25 and 12.5 nM). The level of SOD1 exon skipping following AON transfection was compared to that of a sham control treated and untreated sample.

### Materials and Methods

### Transfection of Fibroblasts

Normal human dermal fibroblasts were propagated according to established techniques with 15,000 cells seeded into 24 well plates in 10% FBS DMEM and incubated at 37°C for 24 hours prior to transfection. All 2'OMethyl PS-AOs were transfected using Lipofectamine 3000 (3 µl per ml of transfection volume) (Life Technologies, Melbourne, Australia), according to manufacturer's protocols, and AO transfected cells incubated for 24 hours.

### Transcript Analysis

RNA was extracted using the MagMAX-96 Total RNA Isolation Kit, including a DNase treatment (Life Technologies), according to the manufacturer's instructions. RT-PCRs were performed using the One-step Superscript III RT-PCR kit with Platinum Taq polymerase (Life Technologies) according to manufacturer's instructions. Products were amplified across SOD1 exons 1 to 5 (Fwd: CGACGAAGGCCGTGTGCGTG (SEQ ID NO: 46), Rev: AGTTAAGGGGCCTCAGACTACA (SEQ ID NO: 47)), with the temperature profile, 55°C for 30 min, 94°C for 2 min, followed by 22 cycles of 94°C for 40 sec, 55°C for 30 sec and 68°C for 1 min.

PCR products were fractionated on 2% agarose gels in Tris-Acetate-EDTA buffer and the images captured on gel documentation system and analysed with Bio1D software (Vilber Lourmat, Eberhardzell, Germany) to quantitate band weight and estimate ratios of full length SOD1 and exon skipped products. Product identity was confirmed by band purification and DNA sequencing as necessary. The efficiency of exon skipping was determined by calculating the percentage of the transcripts with exon(s) skipped compared to the total product generated by RT-PCR.

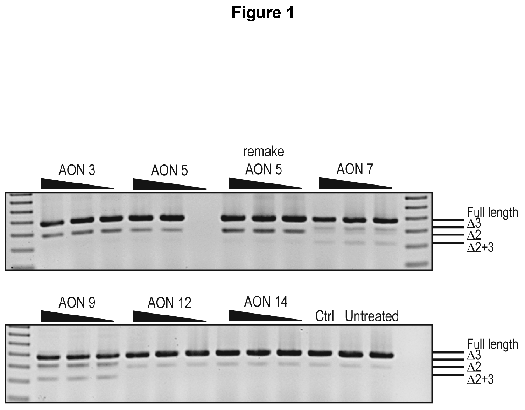

Figure 1 shows RT-PCR analysis of the SOD1 transcript following transfection of 2'OMethyl AONs (50, 25 and 12.5 nM) designed to induce SOD1 exon 2, 3 or 4 skipping.

### Example 4 - Optimisation of AONs

Following AON screening, AONs targeting SOD1 exons 2, 3 and 4 were optimised by micro-walking, and the level of exon skipping following transfection was compared to the original AON sequence, a sham control AON and untreated samples.

The AON sequences were moved up or down stream by 3 to 5 bases to identify the optimal target site, while still maintaining oligomer length. Further optimisation was carried out by varying the AON length between 20 to 25 nucleotides.

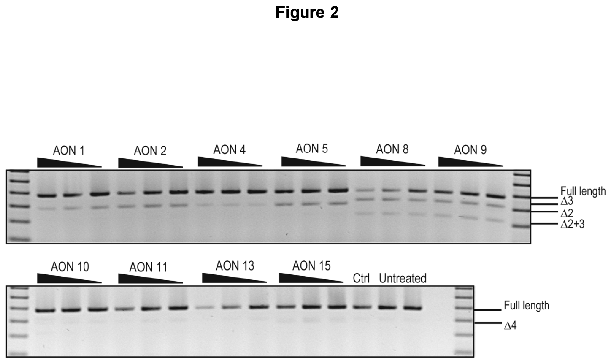

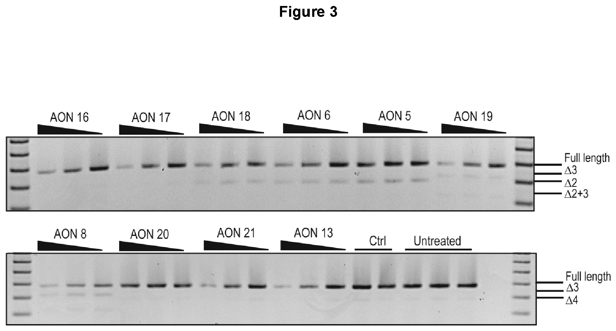

Figures 2 shows RT-PCR analysis of following sequence optimization, compared to the original AON sequence, a sham control and untreated samples. Figure 3 shows the results of further optimization for AONs designed to target the donor site of exon 1, and the levels of full-length SOD1 transcript compared to that of sham control and untreated samples.

AONs 1 to 5 (which correspond to SEQ ID NOs 1 to 5 in Table 1) were designed to induce exon 2 skipping. Of these AONs, AON 5 targeting the exon 2 donor splice site appears to be the most effective at inducing exon 2 skipping, with approximately 20% of transcripts skipping exon 2.

AON 5 was optimized by extending the length by 4 bases, to produce AON 6 (SEQ ID NO: 6). AON 6 does not appear to enhance exon 2 skipping, yet improved the level of knockdown of full length SOD1 (Figure 3). Given the exclusion of exon 2 from the *SOD1* transcript disrupts the reading frame and produces a premature termination codon in exon 4, this is expected to result in nonsense mediated decay and lower levels of full length SOD1 transcript. Micro-walking the AON 6 sequence by shifting the AON 5 bases upstream (AON 18) induces similar levels of exon 2 skipping and SOD1 knockdown to AON 6, indicating this region (Figure 12, Hot Spot 1) to be the optimal target for exon 2 skipping and SOD1 knockdown, targeting SPp30c and SC35 binding sites.

AONs 7 to 10 (which correspond to SEQ ID Nos 7 to 10 in Table 1) were designed to induce exon 3 skipping and following transfection induce low levels of exon 3, and exon 2+3 skipping. AON 8 targeting an exon splice enhancer site appears to be most effective at inducing exon 3 skipping, with a clear decrease in the level of full length SOD1 transcript observed in AON 8 treated samples when compared to that of control treated and untreated samples (Figure 2). Inducing exon 3 skipping is expected to disrupt the reading frame and induce a premature termination codon in exon 4, resulting in nonsense mediated decay, and lower levels of SOD1 transcript.

Optimisation of AON 8 by micro-walking 5 bases upstream (AON 19, corresponding to SEQ ID NO 19 in Table 1) induces similar levels of exon 3 skipping and SOD1 knockdown to that of AON 8 (Figure 3). These AOs target two SRp40 binding sites, indicated as Hot Spot 2 in Figure 12. Furthermore, AONs 8 and 19 induce greater SOD1 knockdown compared to AON 6 (Figure 3), suggesting that exon 3 is the preferred target for inducing nonsense mediated decay and *SOD1* knockdown.

AONs 11 to 15 (which correspond to SEQ ID Nos 11 - 15 in Table 1) were designed to induce exon 4 skipping. AON 13 Is clearly the most effective exon 4 targeting AON to induce SOD1 knockdown (Figure 2). Transcripts lacking exon 4 also have a disrupted reading frame, and a premature stop codon, and therefore nonsense mediated decay would also be expected.

AON optimisation of exon 4 targeting AONs failed to induce exon 4 skipping, yet there is a clear dose dependent decrease in full length SOD1 transcript compared to that of untreated samples. Shifting AON 13 10 bases upstream (AON 21, corresponding to SEQ ID NO 21 in Table 1) was slightly less effective at inducing SOD1 knockdown compared to AON 13. These AONs target an ETR-3 splice enhancer domain, indicated as Hot Spot 3 in Figure 12.

### Example 5 - Transcript Evaluation of AON 6 and AON 8 as PMOs

Following 2'OMethyl AO optimisation, AON 6 and AON 8 were synthesised and evaluated as PMOs. The PMOs were delivered by nucleofection into normal fibroblasts, and then evaluated using RT-PCR as described above.

PMO delivery by nucleofection was performed using a Nucleofection X unit with the Nucleofection P2 kit, using the CA-137 program (Lonza, Melbourne, Australia). PMOs were transfected at 200 µM, 100 µM or 50 µM within the cuvette, supplemented with 5% FBS DMEM and incubated for 24 hours (RNA analysis) and 5 days (protein analysis).

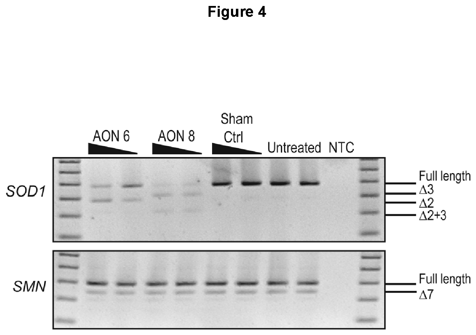

Figure 4 presents the RT-PCR analysis of *SOD1* transcripts following PMO transfection (200 µM and 100 µM). RT-PCR of *SMN* was included as a loading control, and the level of exon skipping following AON transfection is compared to that of sham control and untreated samples.

These PMOs appear to be more effective at inducing full length SOD1 knockdown compared to their 2'OMethyl counterparts. There was a clear dose response in the level of exon skipping and SOD1 knockdown induced by both PMOs. A house keeping PCR was carried out (*SMN*) to ensure the knock down of *SOD1* was specific and confirms equal loading across all lanes.

### Example 6 - Transcript and Protein Analysis of AON 6 and AON 8 as PMOs in normal and SOD1 D90A fibroblasts

Transcript and protein analysis using western blotting was conducted following PMO nucleofection with AON 6 and 8 on normal and patient fibroblasts carrying a *SOD1* D90A mutation.

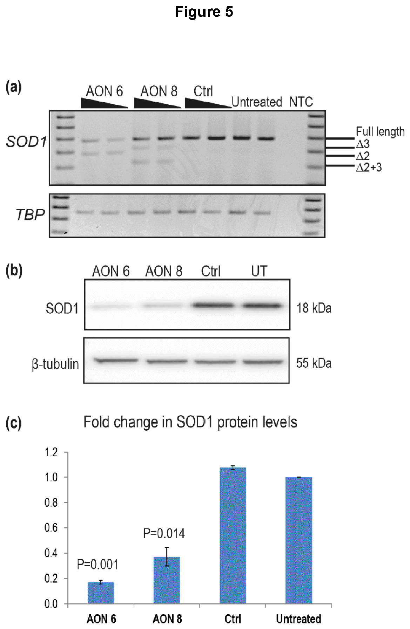

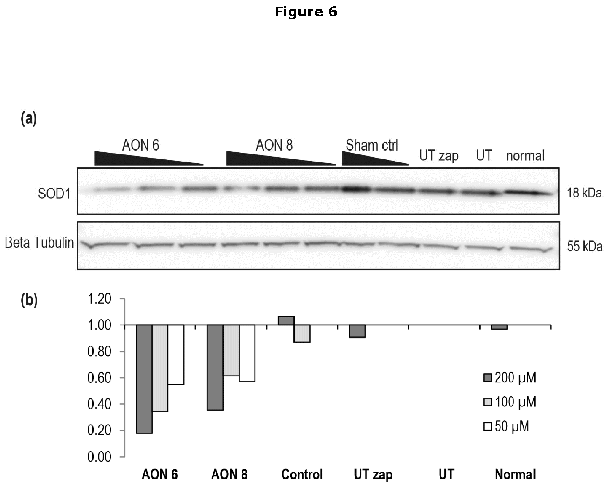

PMOs were delivered by nucleofection at 200 and 100 µM (n=3). The results are presented in Figure 5. PMOs were also delivered by nucleofection at 200, 100 and 50 µM in SOD1 D90A fibroblasts. The results are presented in Figure 6. Nucleofection was conducted as described above.

### Materials and Methods - Western Blotting

Cell lysates were prepared with 125 mM Tris/HCl pH 6.8, 15% SDS, 10% Glycerol, 1.25 µM PMSF (Sigma-Aldrich, NSW, Australia, 1x protease inhibitor cocktail (Sigma-Aldrich) 0.004% bromophenol blue and 2.5 mM dithiothreitol, then sonicated 6 times (1 second pulses). Samples were heated at 94°C for 5 minutes, cooled on ice and centrifuged at 14,000 x g for 2 min before loading onto the gel.

Total protein (30 µg), measured by a Pierce BCA Protein assay kit (Life Technologies), was loaded per sample on a NuPAGE Novex 4-12% BIS/Tris gel (Life Technologies) and separated at 200 V for 55 minutes. Proteins were transferred onto a polyvinylidene fluoride (PVDF) membrane using an iBlot (Life Technologies) with the following transfer protocol: 20 V for 1 min, 23 V for 4 min and 25 V for 2 min. Following blocking for 1 hour at room temperature, the membrane was incubated overnight at 4°C in 5% skim milk powder in 1x TBST containing *SOD1* primary antibody (1:1000, Cell Signalling), and Beta-tubulin primary antibody (1:2500, Life Technologies). Immunodetection was performed using an anti-rabbit HRP secondary antibody (1:10,000, Dako) and the Immobilon HRP chemiluminescent substrate (Merck). Western blot images were captured on a Vilber Lourmat Fusion FX system using Fusion software and Bio- 1D software was used for image analysis.

### Results

Figure 5 shows SOD1 transcript and protein analysis following PMO nucleofection with AON 6 and AON 8 (200 µM and 100 µM for RNA and 200 µM for protein). Figure 5(a) presents RT-PCR of SOD1 transcripts, with TBP transcript analysis included as a loading control; Figure 5(b) presents western blots of SOD1 and β-tubulin (loading control) levels; and Figure 5(c) presents a graph showing the average densitometrical analysis of SOD1 levels in AON treated cells normalised against β-tubulin and expressed as a fold-change compared to *SOD1* levels from untreated fibroblasts. The average was taken from three separate experiments conducted in normal and *SOD1* patient fibroblasts. Sham control transfected and untreated samples were included for comparison to samples from SOD1 transfected fibroblasts. Error bars represent the standard error of the mean, and P values determined using a paired t-test.

Figure 6 shows SOD1 protein analysis following PMO nucleofection with AON 6 and AON 8 in *SOD1* patient fibroblasts carrying a *SOD1* D90A mutation at 200, 100 and 50 µM. Figure 6(a) presents western blots of *SOD1* and β-tubulin (loading control) levels; and Figure 6(b) presents a graph showing the densitometrical analysis of *SOD1* levels in AON treated cells normalised against β-tubulin and expressed as a fold-change compared to *SOD1* levels from untreated fibroblasts. Sham control transfected and untreated samples were included for comparison to samples from SOD1 transfected fibroblasts.

The protein analysis following transfection with AON 6 and AON 8 PMOs showed consistent knockdown of *SOD1* protein levels in both normal and patient fibroblasts carrying a *SOD1* D90A mutation. Figure 5 shows showed an average knock-down of *SOD1* levels by 83% using AON 6 (P=0.001) and 63% using AON 8 (P=0.014) compared to the level in untreated fibroblasts. Figure 6 shows nucleofection of PMOs at 200, 100 and 50 µM in SOD1 D90A fibroblasts showed a clear dose response to the AONs, with the levels of *SOD1* protein decreasing with higher AON concentrations when compared to untreated fibroblasts.

### Example 7 - Differentiation and transfection of SOD1-linked patient induced pluripotent stem cells

*SOD1*-linked patient induced pluripotent stem cells (iPSCs) carrying the A4V, G85R and L144H mutations were differentiated into 70% Hb9 and 80% ChAT positive spinal motor neurons using the protocol published by Du et al. 2015, Nature Comms, 6:6626.

iPS cells were differentiated into neuroepithelial cells in DMEM/F12 and Neurobasal media (1:1) (Life Technologies) supplemented with 0.1 mM ascorbic acid (Life Technologies) 1x Glutamax (Life Technologies), 3 µM CHIR99021 (Torcis), 2 µM DMH-1 (Torcis) and 2 µM SB431542 (Stemgent) for six days. CHIR99021 was reduced to 1 µM and 0.5 µM Purmorphamine (Stemgent) and 0.1 µM retinoic acid (Stemgent) were added for a further six days to differentiate into OLIG2 positive motor neuron progenitor cells. Neural progenitor cells were differentiated for a further six days into MNX1 positive motor neurons in DMEM/F12 Neurobasal medium (1:1) containing 0.5 µM retinoic acid and 0.1 µM purmorphamine. MNX1 positive motor neurons were dissociated with Accumax (eBioscience) into single cells and transfected with SOD1 PMOs using a Neon electroporator (Life Technologies) at 100 µM and 50 µM using the following parameters: 3 pulses at 1300 V for 10 sec. Transfected cells in suspension were replated onto Matrigel coated plates and differentiated into mature ChAT positive motor neurons for a further ten days using 0.1 µM purmorphamine, 0.5 µM retinoic acid and 0.1 µM Compound E (Calbiochem). At all stages, media was replaced every other day. Following transfection and ten-day differentiation protocol, cell culture supernatants were collected and stored at -80°C for LDH analysis (below) and cells harvested for RNA and protein analysis as above.

### Example 8 - Transfection of AON 6 and AON 8 PMOs into iPSC derived motor neurons

PMO electroporation with AON 6 and AON 8 into *SOD1*-linked patient induced pluripotent stem cell derived motor neurons was performed as above (100 and 50µM).

### Example 9 - Transcript and Protein Analysis of AON 6 and AON 8 as PMOs in iPSC derived motor neurons

AON 6 and AON 8 PMOs evaluated in SOD1-linked ALS patient induced pluripotent stem cell (iPSC)-derived motor neurons, carrying SOD1 A4V, G85R and L144H mutations. Transcript analysis was performed using RT-PCR as described above, and protein analysis was performed using western blotting as described above.

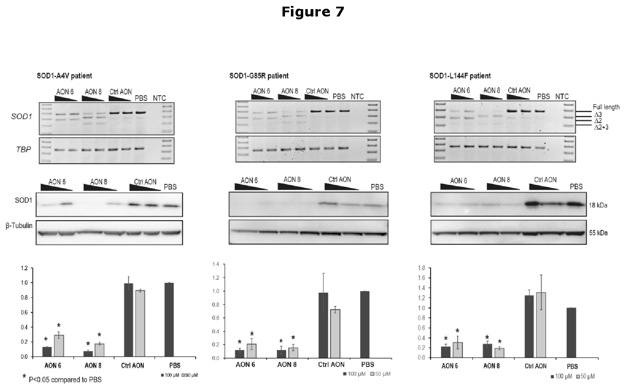

The results are set out in Figure 7. Figure 7(a) presents RT-PCR across SOD1 transcripts with *TBP* transcript analysis included as a loading control, Figure 7(b) presents western blots of SOD1 and β-tubulin (loading control) levels; and Figure 7(c) presents graph showing the densitometry analysis of *SOD1* levels in AON treated cells normalised against β-tubulin and expressed as a fold-change compared to SOD1 levels in motor neurons treated with PBS. Sham control transfected samples were included for comparison to samples from *SOD1* AON transfected motor neurons.

At both concentrations (100 and 50 µM), the AON 6 and AON 8 PMOs both induced significant knockdown of SOD1 protein levels in all three patient cell strains. AON 8 was the most consistent and effective PMO in motor neurons, with up to 93% knockdown induced at the higher concentration (P=0.001) in all three cell strains.

### Example 10 - Assessment of cell viability following PMO transfection in SOD1A4V patient motor neurons

Following PMO transfection in *SOD1*^{A24V} patient motor neurons (100 and 50 µM), Lactate dehydrogenase (LDH) levels were assessed in the cell culture supernatant as a measure of cell death.

LDH levels were measured in supernatants using an LDH CytoTox 96 Cytotoxicity assay kit (Promega, G1780) performed according to the manufacturer's protocol. LDH levels in supernatants from AON transfected cells were normalised to levels detected in supernatants from PBS control transfected cells.

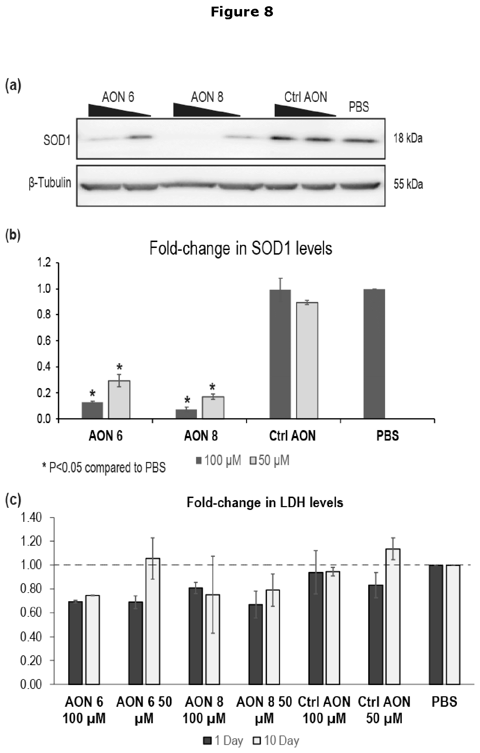

The results are presented in Figure 8. Figure 8(a) and (b) show western blots and densitometry as in Figure 7, and Figure 8(c) is a graph of LDH levels from cell culture supernatants collected 1 and 10 days following PMO transfection, whereby the LDH levels in *SOD1* AON transfected cells were normalised to the levels in PBS treated cells. Both AON 6 and AON 8 appear to improve cell viability, with lower levels of LDH observed in supernatants from SOD1-PMO transfected cells when compared to the PBS treated cells.

### Example 11 - Assessment of oxidative stress in SOD1 D90A fibroblasts transfected with AONs

The effect of SOD1 protein suppression on intracellular levels of reactive oxygen species (ROS) following hydrogen peroxide injury to induce oxidative stress in PMO transfected (50 and 25 µM) *SOD1*^{D90A} fibroblasts was evaluated.

The PMO electroporation was performed with a Neon electroporator with resuspension buffer R and the following parameters: 3 pulses at 1300 V for 10 sec (Life Technologies). PMOs were transfected at 50 µM, 25 µM or 10 µM within the Neon tip, supplemented with 5% FBS DMEM and incubated for 24 hours (RNA analysis) and 5 days (protein analysis).

*SOD1*^{D90A} fibroblasts were transfected with AONs using Neon electroporation as described above. Four days following AON transfection, cells were treated with 5 mM hydrogen peroxide (Sigma-Aldrich) to induce oxidative stress, incubated for 30 min (37°C), then hydrogen peroxide removed and replaced with fresh media. Fibroblasts were incubated for an additional 24 hours prior to trypsinising and cell pellets collected equally into two tubes for total protein analysis by BCA and for analysis of reactive oxygen species (ROS). Cell pellets for ROS analysis were resuspended in PBS and analysed for intracellular ROS using a DCF ROS/RNS Assay kit (Abcam, Ab238535) to detect DCF Fluorescence. Each sample was run in duplicate and the assay performed according to the manufacturer's protocol. Levels of intracellular ROS were normalised to total protein.

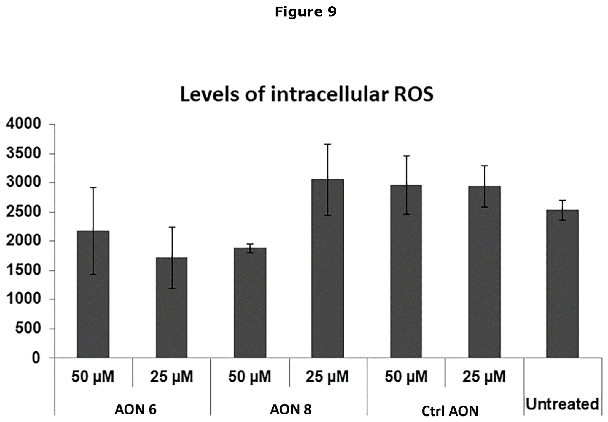

The results are presented in Figure 9, which shows ROS levels in fibroblasts transfected with AON 6 and AON 8 compared to levels in untreated fibroblasts. ROS levels were normalised to total protein by BCA analysis. Knockdown of mutant SOD1 protein appears to improve cellular response to hydrogen peroxide with AON 6 and AON 8 PMO transfected cells having lower levels of intracellular ROS compared to control PMO transfected and untreated fibroblasts.

### Example 12 - Comparison of AON 8 PMO with RNase H MOE AON

AON 8 PMO efficacy was compared to the SOD1 targeting RNase H phosphorothioate 2'-O-Methoxyethyl (MOE) AON, described in (McCampbell, et al, J Clin Invest., 2018. 128(8)), following transfection into *SOD1*^{D90A} fibroblasts. Transfection occurred through Neon electroporation, and protein analysis was conducted using Western blotting as described above.

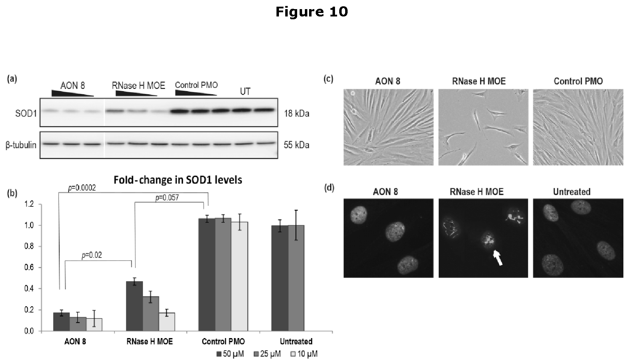

Figure 10 shows a comparison of the effect of AON 8 PMO and an RNase H phosphorothioate AON on SOD1 protein levels and cellular toxicity following electroporation (50, 25 and 10 µM) into *SOD1*D^{90A} fibroblasts. Figure 10(a) presents western blots of *SOD1* and β-tubulin (loading control) levels; Figure 10(b) presents a graph showing the densitometry analysis of SOD1 levels in AON treated cells normalised against β-tubulin and expressed as a fold-change compared to SOD1 levels untreated fibroblasts; Figure 10(c) presents phase contrast images of AON transfected fibroblasts (50 µM) prior to lysis; and Figure 10(d) presents immunofluorescent images of the off-target effects to paraspeckle proteins following AON transfection, staining for NONO (indicated by white arrows) and counterstained with Hoechst for nuclei detection.

AON 8 appears to be the more consistent and effective AO at all concentrations, inducing 82% SOD1 knockdown at the higher dose compared to the control PMO (p=0.0002) and was more effective than the RNase H MOE AON (p=0.02). AON 8 had no negative impact on cellular viability and density, while the RNase H MOE AON caused significant cell death at the higher dose (Figure 10(c)), likely accounting for the reverse dose-response observed in SOD1 levels from the RNase H AON.

Previous reports have shown toxic effects due off-target binding of phosphorothioate AOs to paraspeckle proteins causing protein sequestration into aggregates, leading to global effects to the transcriptome, influencing RNA processing, splicing and transcription, among other processes (Flynn et al, BioRxiv, 2018, 446773; Shen et al, Nucleic Acids Res 42(13):880 8648-8662). Immunostaining for the paraspeckle protein NONO (Figure 10(d)) revealed off-target protein binding of the RNase H MOE AON with NONO aggregates within the nucleus, while the AON 8 PMO did not cause off-target protein binding. Taken together, this experiment suggests that the AON 8 PMO may be more effective and safer than the RNase H MOE AON.

### Example 13 - In Vivo assessment of AON 6 and AON 8

Three transgenic *SOD1*^{G93A} mice were treated with AON 6 or AON 8, and brain and spinal tissues were analysed for *SOD1* expression. AON 6 and AON 8 PMOs and a control PMO were delivered intracerebroventricularly (ICV) (100 nmol) to high copy number transgenic *SOD1*^{G93A} mice at P60 (n=3) and sacrificed at P45.

Transgenic SOD1G93A mice (B6.Cg-Tg (SOD1*G93A)1Gur/J line, stock number 004435, RRID:IMSR _JAX:004435) were purchased from the Jackson Laboratory (Bar Harbor, ME, USA). The colony was maintained on a C57BL/6 background at the Florey Institute of Neuroscience and Mental Health Core Animal Services under specific pathogen-free conditions. Experimental animals were group housed in microisolator caging under standard 12-hr light-dark conditions with access to food and water ad libitum.

High SOD1 expressing mice were used for AON evaluation. 30 day old mice were anaesthetised by isoflurane and administered 100 nmol of AON by intracerebroventricular bolus injection using a stereotactic device. Mice were sacrificed at 45 days and the brain and spinal cord harvested, snap frozen and stored at -80°C. Tissues were cryosectioned using a cryostat (Leica) and sections lysed for RNA and protein analysis. For protein analysis, cryosections were weighed and lysed using home-made protein lysis buffer as described above, with 150 µl of buffer/4.5 mg of tissue. Tissues were sonicated and analysed for SOD1 expression as described. Cryosections for RNA analysis were lysed in lysis buffer provided with the MagMax total RNA isolation kit (Life Technologies) and analysed as described.

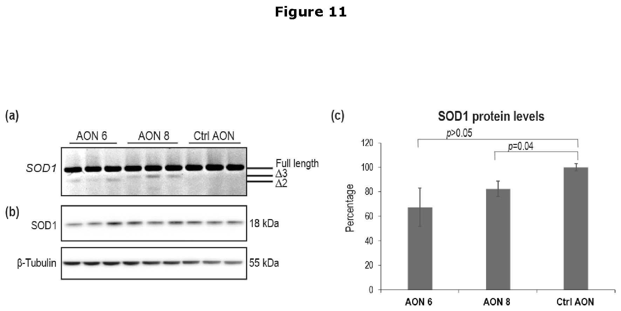

*SOD1* transcript and protein expression were analysed within brain lysates. Transcript analysis was conducted using RT-PCR as described above and protein analysis was conducted using Western blotting as described above. The results are presented in Figure 11. Figure 11(a) presents RT-PCR analysis of SOD1 transcripts; 11(b) presents western blots of *SOD1* and β-tubulin (loading control) protein levels; and 11(c) presents densitometry analysis of *SOD1* protein levels in *SOD1* PMO treated mice, normalised to β-tubulin, and compared to control PMO treated mice.

Low levels of SOD1 exon skipping was observed in mice treated with both PMOs. Western blot analysis showed an average 33% reduction in *SOD1* protein in AON 6 treated mice. AON 8 treated mice showed a statistically significant 20% reduction in *SOD1* protein compared to control treated mice (p=0.04). The difference in PMO efficacy *in vitro* and *in vivo* is likely due to splicing machinery differences between human cells and the transgenic mouse model.

### Patentin SEQUENCE Information

SEQUENCE LISTING

<110>  Perron Institute for Neurological and Translational Science
       Limited

<120>  Treatment for SOD1 Associated Disease

<130>  280776

<160>  47

<170>  PatentIn version 3.5

<210>  1
<211>  25
<212>  RNA
<213>  Homo sapiens

<400>  1
guccauuacu uuccuuuaag aaaag                                          25

<210>  2
<211>  25
<212>  RNA
<213>  Homo sapiens

<400>  2
guccuuuaau gcuuccccac accuu                                          25

<210>  3
<211>  22
<212>  RNA
<213>  Homo sapiens

<400>  3
caggcuucag ucaguccuuu aa                                             22

<210>  4
<211>  26

<212>  RNA
<213>  Homo sapiens

<400>  4
acucaugaac auggaaucca ugcagg                                    26



<210>  5
<211>  21
<212>  RNA
<213>  Homo sapiens

<400>  5
acacccaccu gcuguauuau c                                         21



<210>  6
<211>  25
<212>  RNA
<213>  Homo sapiens

<400>  6
aacacccacc ugcuguauua ucucc                                     25



<210>  7
<211>  23
<212>  RNA
<213>  Homo sapiens

<400>  7
uaaagugagg accugcacug gua                                       23



<210>  8
<211>  25
<212>  RNA
<213>  Homo sapiens

<400>  8
auccuuuggc ccaccguguu uucug                                     25



<210>  9
<211>  21

<212>  RNA
<213>  Homo sapiens

<400>  9
cauccuuugg cccaccgugu u                                                    21



<210>  10
<211>  25
<212>  RNA
<213>  Homo sapiens

<400>  10
guuaccucuc uucauccuuu ggccc                                                25



<210>  11
<211>  25
<212>  RNA
<213>  Homo sapiens

<400>  11
ccaacaugcc uaauaaugaa aaagc                                                25



<210>  12
<211>  23
<212>  RNA
<213>  Homo sapiens

<400>  12
uuugucagca gucacauugc cca                                                  23



<210>  13
<211>  25
<212>  RNA
<213>  Homo sapiens

<400>  13
caauagacac aucggccaca ccauc                                                25



<210>  14
<211>  23

<212>  RNA
<213>  Homo sapiens

<400>  14
ugaugcaaug gucuccugag agu                                                    23

<210>  15
<211>  25
<212>  RNA
<213>  Homo sapiens

<400>  15
uccuuuuaug aaaacuuacc accag                                                  25

<210>  16
<211>  25
<212>  RNA
<213>  Homo sapiens

<400>  16
ugaugaugcc cugcacuggg ccguc                                                  25

<210>  17
<211>  25
<212>  RNA
<213>  Homo sapiens

<400>  17
cuugccuucu gcucgaaauu gauga                                                  25

<210>  18
<211>  25
<212>  RNA
<213>  Homo sapiens

<400>  18
ccaccugcug uauuaucucc aaacu                                                  25

<210>  19
<211>  25

<212>  RNA
<213>  Homo sapiens

<400>  19
uuggcccacc guguuuucug gauag                                    25

<210>  20
<211>  25
<212>  RNA
<213>  Homo sapiens

<400>  20
auuaggcaug uuggagacuu gggca                                    25

<210>  21
<211>  25
<212>  RNA
<213>  Homo sapiens

<400>  21
ugcugacaaa gauggugugg ccgau                                    25

<210>  22
<211>  25
<212>  DNA
<213>  Homo sapiens

<400>  22
gtccattact ttcctttaag aaaag                                    25

<210>  23
<211>  25
<212>  DNA
<213>  Homo sapiens

<400>  23
gtcctttaat gcttccccac acctt                                    25

<210>  24
<211>  22

<212>  DNA
<213>  Homo sapiens

<400>  24
caggcttcag tcagtccttt aa                                          22

<210>  25
<211>  26
<212>  DNA
<213>  Homo sapiens

<400>  25
actcatgaac atggaatcca tgcagg                                      26

<210>  26
<211>  21
<212>  DNA
<213>  Homo sapiens

<400>  26
acacccacct gctgtattat c                                           21

<210>  27
<211>  25
<212>  DNA
<213>  Homo sapiens

<400>  27
aacacccacc tgctgtatta tctcc                                       25

<210>  28
<211>  23
<212>  DNA
<213>  Homo sapiens

<400>  28
taaagtgagg acctgcactg gta                                         23

<210>  29
<211>  25

<212>  DNA
<213>  Homo sapiens

<400>  29
atcctttggc ccaccgtgtt ttctg                                        25



<210>  30
<211>  21
<212>  DNA
<213>  Homo sapiens

<400>  30
catcctttgg cccaccgtgt t                                            21



<210>  31
<211>  25
<212>  DNA
<213>  Homo sapiens

<400>  31
gttacctctc ttcatccttt ggccc                                        25



<210>  32
<211>  25
<212>  DNA
<213>  Homo sapiens

<400>  32
ccaacatgcc taataatgaa aaagc                                        25



<210>  33
<211>  23
<212>  DNA
<213>  Homo sapiens

<400>  33
tttgtcagca gtcacattgc cca                                          23



<210>  34
<211>  25

<212>  DNA
<213>  Homo sapiens

<400>  34
caatagacac atcggccaca ccatc                                          25

<210>  35
<211>  23
<212>  DNA
<213>  Homo sapiens

<400>  35
tgatgcaatg gtctcctgag agt                                            23

<210>  36
<211>  25
<212>  DNA
<213>  Homo sapiens

<400>  36
tccttttatg aaaacttacc accag                                          25

<210>  37
<211>  25
<212>  DNA
<213>  Homo sapiens

<400>  37
tgatgatgcc ctgcactggg ccgtc                                          25

<210>  38
<211>  25
<212>  DNA
<213>  Homo sapiens

<400>  38
cttgccttct gctcgaaatt gatga                                          25

<210>  39
<211>  25

<212>  DNA
<213>  Homo sapiens

<400>  39
ccacctgctg tattatctcc aaact                                    25

<210>  40
<211>  25
<212>  DNA
<213>  Homo sapiens

<400>  40
ttggcccacc gtgttttctg gatag                                    25

<210>  41
<211>  25
<212>  DNA
<213>  Homo sapiens

<400>  41
attaggcatg ttggagactt gggca                                    25

<210>  42
<211>  25
<212>  DNA
<213>  Homo sapiens

<400>  42
tgctgacaaa gatggtgtgg ccgat                                    25

<210>  43
<211>  9
<212>  DNA
<213>  Homo sapiens

<400>  43
aacacccac                                    9

<210>  44
<211>  16

<212>  RNA
<213>  Homo sapiens

<400>  44
cugcuguauu aucucc                                                    16



<210>  45
<211>  16
<212>  DNA
<213>  Homo sapiens

<400>  45
ctgctgtatt atctcc                                                    16



<210>  46
<211>  20
<212>  DNA
<213>  Homo sapiens

<400>  46
cgacgaaggc cgtgtgcgtg                                                20



<210>  47
<211>  22
<212>  DNA
<213>  Homo sapiens

<400>  47
agttaagggg cctcagacta ca                                             22

### Itemized list of embodiments

1. An isolated or purified antisense oligonucleotide targeted to a nucleic acid molecule encoding superoxide dismutase 1 pre-mRNA, the AON has a nucleobase sequence that is:
   a. selected from the list comprising SEQ ID NO: 1 to SEQ ID NO: 42 inclusive, or
   b. a sequence that is of sufficient sequence complementarity to a target RNA to induce exon skipping wherein said sequence is complementary to at least 8 or more contiguous nucleobases in a target *SOD1* pre-mRNA to which SEQ ID NO: 1 to SEQ ID NO: 42 inclusive also bind, and
   c. wherein the AON inhibits the expression of human *SOD1.*
2. An isolated or purified antisense oligonucleotide targeted to a nucleic acid molecule encoding superoxide dismutase 1 pre-mRNA, wherein the antisense oligonucleotide has a nucleobase sequence selected from the list comprising SEQ ID NO: 1 to SEQ ID NO: 42 inclusive, and wherein the antisense oligonucleotide inhibits the expression of human *SOD1.*
3. The antisense oligonucleotide of item 1 or 2 that induces alternative splicing of *SOD1* pre-mRNA through exon skipping.
4. The antisense oligonucleotide of items 1 to 3 that is a phosphorodiamidate morpholino oligomer.
5. The antisense oligonucleotide of item 4 that is a peptide-phosphorodiamidate morpholino oligomer conjugate.
6. The antisense oligonucleotide of items 1 to 3 that is selected from the list of SEQ ID NO: 6, 8, 13, 27, 29 or 34.
7. A method of inducing alternative splicing of SOD1 pre-mRNA, the method comprising the steps of:
   a) providing one or more of the AONs according to any one of items 1 to 6; and
   b) allowing the oligomer(s) to bind to a target nucleic acid site.
8. A pharmaceutical, prophylactic, or therapeutic composition to treat, prevent or ameliorate the effects of a disease associated with mutations or misfolding in *SOD1,* the composition comprising:
   a) one or more antisense oligonucleotides according to any one of items 1 to 6; and
   b) one or more pharmaceutically acceptable carriers and/or diluents.
9. The pharmaceutical composition of item 8 wherein the disease associated with mutations or misfolding in *SOD1* is ALS.
10. A pharmaceutical, prophylactic, or therapeutic composition to treat, prevent or ameliorate the effects of ALS, the composition comprising:
   a) one or more antisense oligonucleotides according to any one of items 1 to 6; and
   b) one or more pharmaceutically acceptable carriers and/or diluents.
11. A method of treating, preventing or ameliorating the effects of a disease associated with mutations or misfolding in *SOD1,* the method comprising the step of:
   a) administering to the subject an effective amount of one or more antisense oligonucleotides or pharmaceutical composition comprising one or more antisense oligonucleotides according to any one of items 1 to 6, 8 or 10.
12. The method of treatment of item 11 wherein the disease associated with mutations or misfolding in *SOD1* is ALS.
13. A method of treating, preventing or ameliorating the effects of ALS, the method comprising the step of:
   a) administering to the subject an effective amount of one or more antisense oligonucleotides or pharmaceutical composition comprising one or more antisense oligonucleotides according to any one of items 1 to 6, 8 or 10.
14. A method for treating, preventing or ameliorating the effects of ALS, in patients identified by a biomarker, the method comprising the step of:
   a) testing a subject for the presence of a biomarker associated with ALS patients likely to respond to SOD1 suppression; and
   b) if the subject is found to express the biomarker, administering to the subject an effective amount of one or more antisense oligonucleotides or pharmaceutical composition comprising one or more antisense oligonucleotides according to any one of items 1 to 6, 8 or 10.
15. An expression vector comprising the antisense oligonucleotide according to any one of items 1 to 6.
16. The use of purified and isolated antisense oligonucleotides according to any one of items 1 to 6, for the manufacture of a medicament to treat, prevent or ameliorate the effects of a disease associated with mutations or misfolding in *SOD1.*
17. The use of purified and isolated antisense oligonucleotides according to any one of items 1 to 6, to treat, prevent or ameliorate the effects of a disease associated with mutations or misfolding in *SOD1.*
18. The use of items 16 or 17 wherein the disease associated with mutations or misfolding in *SOD1* is ALS.
19. The use of purified and isolated antisense oligonucleotides according to any one of items 1 to 6, to treat, prevent or ameliorate the effects of ALS.
20. The use of purified and isolated antisense oligonucleotides according to any one of items 1 to 6, for the manufacture of a medicament to treat, prevent or ameliorate the effects of ALS.
21. A kit to treat, prevent or ameliorate the effects of a disease associated with mutations or misfolding in *SOD1* in a subject, which kit comprises at least an antisense oligonucleotide according to any one of items 1 to 6, packaged in a suitable container, together with instructions for its use.
22. The kit of item 21 wherein the disease associated with mutations or misfolding in *SOD1* is ALS.
23. A kit to treat, prevent or ameliorate the effects of ALS, which kit comprises at least an antisense oligonucleotide according to any one of items 1 to 6, packaged in a suitable container, together with instructions for its use.

## Claims

1. An isolated or purified antisense oligonucleotide (AON) targeted to a nucleic acid molecule encoding superoxide dismutase 1 (SOD1) pre-mRNA, wherein the AON has a nucleobase sequence comprising:
a. SEQ ID NO: 8 or SEQ ID NO: 29, or
b. a sequence that is of sufficient sequence complementarity to a target SOD1 RNA to induce exon skipping wherein said sequence is complementary to at least 21 or more contiguous nucleobases in a target *SOD1* pre-mRNA to which SEQ ID NO: 8 or SEQ ID NO: 29 also bind, and
wherein the AON of a) or b) inhibits the expression of human *SOD1.*

2. An isolated or purified antisense oligonucleotide targeted to a nucleic acid molecule encoding superoxide dismutase 1 pre-mRNA, wherein the antisense oligonucleotide has a nucleobase sequence comprising SEQ ID NO: 8 or SEQ ID NO: 29, and wherein the antisense oligonucleotide inhibits the expression of human *SOD1* through exon skipping.

3. The antisense oligonucleotide of claim 1 or 2 that induces alternative splicing of *SOD1* pre-mRNA through exon skipping to induce nonsense mediated decay of *SOD1.*

4. The antisense oligonucleotide according to any one of claims 1 to 3 that is a phosphorodiamidate morpholino oligomer.

5. The antisense oligonucleotide of claim 4 that is a peptide-phosphorodiamidate morpholino oligomer conjugate.

6. The antisense oligonucleotide according to any one of claims 1 to 3 comprising SEQ ID NOs: 8, or 29, or uracil or thymine derivatives, respectively, of the same target site.

7. A method of inducing alternative splicing of *SOD1* pre-mRNA *ex vivo,* the method comprising the steps of:
a) providing one or more of the AONs according to any one of claims 1 to 6; and
b) allowing the one or more of the AONs to bind to a target SOD1 nucleic acid site enriched for exon splice enhancer motifs.

8. An isolated or purified antisense oligonucleotide according to any one of claims 1 to 6 for use in inducing alternative splicing of *SOD1* pre-mRNA comprising providing one or more of said AONs and allowing the one or more of the AONs to bind to a target nucleic acid site enriched for exon splice enhance motifs.

9. A composition comprising:
a) one or more antisense oligonucleotides according to any one of claims 1 to 6; and
b) one or more pharmaceutically acceptable carriers and/or diluents.

10. An isolated or purified antisense oligonucleotide according to any one of claims 1-6, or a composition according to claim 9, for use in treating, preventing or ameliorating the effects of a disease associated with mutations or misfolding in *SOD1.*

11. The antisense oligonucleotide or the composition for use according to claim 10 wherein the disease associated with mutations or misfolding in *SOD1* is ALS.

12. An isolated or purified antisense oligonucleotide according to any one of claims 1-6, or a composition according to claim 9, for use in treating, preventing or ameliorating the effects of ALS in a subject identified by a molecular biomarker, including a genomic, transcriptomic or protein signature, such as carriage of a genetic marker in the *SCAF4* or *SQSTM1* gene, or presence of misfolded SOD1 protein within the CSF,
wherein the subject is first tested for the presence of a molecular biomarker associated with ALS patients likely to respond to SOD1 suppression.

13. An expression vector comprising the antisense oligonucleotide according to any one of claims 1 to 6.

14. A kit to treat, prevent or ameliorate the effects of a disease associated with mutations or misfolding in *SOD1* in a subject, which kit comprises at least an antisense oligonucleotide according to any one of claims 1 to 6, packaged in a suitable container, together with instructions for its use.

15. The kit of claim 14 wherein the disease associated with mutations or misfolding in *SOD1* is ALS.
